(19) Europäisches Patentamt
European Patent Office
Office européen des brevets

(11) **EP 4 640 751 A1**

(12) **EUROPEAN PATENT APPLICATION**
published in accordance with Art. 153(4) EPC

(43) Date of publication:
**29.10.2025 Bulletin 2025/44**

(21) Application number: **23906492.6**

(22) Date of filing: **06.11.2023**

(51) International Patent Classification (IPC):
*C08J 3/24* (2006.01)          *C09D 5/16* (2006.01)
*A61Q 1/00* (2006.01)          *A61Q 19/10* (2006.01)
*C08K 5/09* (2006.01)          *C08L 5/04* (2006.01)
*C08L 101/16* (2006.01)        *C09D 201/00* (2006.01)
*C09D 7/65* (2018.01)          *A61K 8/73* (2006.01)

(52) Cooperative Patent Classification (CPC):
A61K 8/73; A61Q 1/00; A61Q 19/10; C08J 3/24;
C08K 5/09; C08L 5/04; C08L 101/16; C09D 5/16;
C09D 7/65; C09D 201/00

(86) International application number:
**PCT/JP2023/039849**

(87) International publication number:
**WO 2024/135121 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.12.2022 JP 2022204588**

(71) Applicant: **Nisshinbo Holdings Inc.
Tokyo 103-8650 (JP)**

(72) Inventors:
• **HASHIBA Toshifumi
Chiba-shi, Chiba 267-0056 (JP)**
• **HAYAKAWA Kazutoshi
Chiba-shi, Chiba 267-0056 (JP)**
• **UEMURA Naohiro
Chiba-shi, Chiba 267-0056 (JP)**

(74) Representative: **Mewburn Ellis LLP
Aurora Building
Counterslip
Bristol BS1 6BX (GB)**

(54) **MARINE BIODEGRADABLE POLYMER PARTICLE GROUPS AND PRODUCTION METHOD FOR SAME**

(57) Marine biodegradable polymer particle groups according to the present invention comprise a polymer compound obtained by using two or more at least divalent metal cations to crosslink a water-soluble polymeric polyvalent anion that has a monovalent anionic substituent and includes at least a compound derived from alginic acid. The marine biodegradable polymer particle groups satisfy conditions (1)-(4). (1) The at least divalent metal cations are 3-30 mass% of the particle groups. (2) The difference between the maximum value and the minimum value of the atomic radii of the metal elements of the at least divalent metal cations is at least 15 Å. (3) The at least divalent metal cations of metal elements that have atomic radii of at least 150 Å are at least 25 mass% of all the at least divalent metal cations. (4) The water absorption of the particle groups is less than 300 mL/100 g.

EP 4 640 751 A1

**Description**

TECHNICAL FIELD

The present invention relates to marine biodegradable polymer particles and a method for producing the same.

BACKGROUND ART

Environmental pollution (marine contamination) by microplastics and the harmful effects of microplastics on ecosystems have become a problem in recent years, and so a variety of efforts to reduce their environmental impact have been launched. Noteworthy among these efforts is the development and widespread adoption of biodegradable resins. Ordinary biodegradable resins exhibit high biodegradability in environments such as soils and sludge where there is an abundance of microorganisms that carry out decomposition. However, decomposition does not readily take place in environments such as the oceans where the concentration of microorganisms is extremely low. Very few resins at present are biodegradable in the ocean.

Microparticles are used as an important material in key industries. Synthetic resins have been used up until now as a starting material for microparticles, but environmental pollution (marine contamination) and adverse ecological impacts have become problems, and so efforts are starting to be made to reduce the generation of microplastics.

Although there is interest in the use of naturally occurring polymers such as cellulose as starting materials for making microparticles, cellulose absorbs water easily and has a relatively high swellability, which makes it undesirable in terms of dimensional stability and also tactile feel and moldability. Moreover, even though cellulose has a high degradability on land such as in the soil, the solubility and degradability of cellulose in the marine environment are low.

Even among natural polymers, alginic acid, which is a polymer derived from seaweed, breaks down relatively quickly in the ocean due to degradation by marine microorganisms and by enzymes and the like released from seaweeds, shellfish, etc.

As described in Non-Patent Document 1, the swellability of alginic acid can be suppressed by calcium crosslinking, enabling the special features of alginic acid to be put to maximal use in cosmetics such as antiperspirants that require moisture absorbing and releasing properties, which is highly advantageous. However, in base makeup, point makeup and other uses where a softness and tactile feel like that of existing plastics are regarded as important, the highly crosslinked structure called an "egg-box" structure gives rise to adverse effects.

Patent Document Nos. 1 and 2 disclose art that uses hydrophobizing agents to improve the tactile feel. Although these do achieve certain advantageous effects, there exists a desire for improvement in terms of softness, smoothness, flexibility and texture.

Similarly, in the area of industrial materials in general, such as coatings and molded articles, given environmental concerns such as marine contamination, there exists a desire for even more useful materials to take the place of existing polymer beads used as texture enhancers or for imparting optical properties.

PRIOR ART DOCUMENTS

PATENT DOCUMENTS

    Patent Document 1: JP-A 2020-125256
    Patent Document 2: JP-A 2021-195321

NON-PATENT DOCUMENT

Non-Patent Document 1: Sato, T., et al.: "Development of calcium alginate microparticles and expansion of their use into cosmetics," Sen'i to Kogyo, 20, No. 1, pp. 20-26 (1996).

SUMMARY OF INVENTION

TECHNICAL PROBLEM

The present invention was arrived at in light of the above circumstances. The object of this invention is to provide practically useful marine biodegradable polymer particles which, by resolving difficulties with swellability, dimensional stability, tactile feel, optical properties, moldability and the like associated with naturally occurring ingredients, can be used in place of polymer beads in cosmetics, etc. and solves the microplastics problem caused by existing general-purpose polymer ingredients.

SOLUTION TO PROBLEM

The inventors have conducted intensive investigations in order to achieve the above object. As a result, they have discovered that, in polymer particles obtained by metal cation crosslinking, by appropriately adjusting the type and content of metal cations used in crosslinking, the crosslinked structure and the crosslink density, the particles can be imparted with softness, smoothness, texture, and optical properties such as the ability to scatter ultraviolet (UV) light while at the same time stabilizing the structure. The inventors have also found that by using such polymer particles in combination with a resin, particularly a biodegradable resin, this material is the first to incur primary decomposition in seawater and has: (1) the effect of forming holes in the resin material, increasing the specific surface area of the resin and stimulating the growth of microorganisms which carry out decomposition, and (2) the effect of, owing to primary decomposition, accelerating secondary decomposition, i.e., biodegradation by microorganisms. As a result, the biodegradation of resin materials in the ocean can be accelerated, enabling the microplastics problem to be solved. This discovery ultimately led to the present invention.

Accordingly, this invention provides the following marine biodegradable polymer particles and method for producing the same.

1. Marine biodegradable polymer particles containing a polymer compound in which water-soluble polymeric polyvalent anions having monovalent anionic substituents are crosslinked through two or more types of at least divalent metal cations, the water-soluble polymeric polyvalent anions including at least one anion derived from alginic acid,

wherein the marine biodegradable polymer particles satisfy conditions (1) to (4) below:

(1) the content of the at least divalent metal cations included in the particles is from 3 to 30 wt%;
(2) the difference between the largest and smallest atomic radii of the metal elements making up the at least divalent metal cations is 15 Å or more;
(3) the content of the at least divalent metal cations derived from metal elements having an atomic radius of 150 Å or more is at least 25 wt% of all the at least divalent metal cations; and
(4) the particles have a water absorption of less than 300 mL/100 g.

2. The marine biodegradable polymer particles of 1 above, wherein the monovalent anionic substituents are carboxylate anions.

3. The marine biodegradable polymer particles of 1 or 2 above, wherein the water-soluble polymeric polyvalent anions are derived from a polysaccharide.

4. The marine biodegradable polymer particles of any of 1 to 3 above, wherein the difference between the largest and smallest atomic radii of the metal elements making up the at least divalent metal cations is 100 Å or less.

5. The marine biodegradable polymer particles of any of 1 to 4 above, wherein the atomic radii of the metal elements making up the at least divalent metal cations are from 80 to 220 Å.

6. The marine biodegradable polymer particles of any of 1 to 5 above, wherein at least one of the at least divalent metal cations is a beryllium, magnesium, calcium, strontium, barium, zinc or aluminum ion.

7. The marine biodegradable polymer particles of 6 above, wherein at least one of the at least divalent metal cations is a calcium or strontium ion.

8. The marine biodegradable polymer particles of any one of 1 to 7 above which further satisfy condition (5) below:
(5) the 10% compressive strength $K_{10}$ at 10% displacement of the particle diameter is from 1 to 1,000 MPa.

9. The marine biodegradable polymer particles of any of 1 to 8 above which further satisfy condition (6) below:
(6) the melting temperature is at least 150°C.

10. A method for producing marine biodegradable polymer particles, which method includes the step of crosslinking water-soluble polymeric polyvalent anions derived from a water-soluble anionic polymer containing at least a monovalent salt of alginic acid and having monovalent anionic substituents within:

(A) a medium in which particles of the water-soluble anionic polymer have been dispersed to a concentration of at least 5 wt%,
(B) a medium in which, using water and an oily medium, the water-soluble anionic polymer has been suspended or emulsified in water to a concentration of at least 5 wt%, or
(C) a medium in which the water-soluble anionic polymer has been hydrophilized or dissolved to a concentration of at least 5 wt%

by using as crosslinking agents two or more salt compounds containing at least divalent metal cations;
wherein the crosslinking reaction is carried out by adding the two or more salt compounds a plurality of times in divided portions, such that the difference between the largest and smallest atomic radii of the metal elements making up the at least divalent metal cations included in the salt compound is 15 Å or more, and the content of the at least divalent metal cations derived from metal elements having an atomic radius of 150 Å or more is at least 25 wt% of all the at least divalent metal cations.

11. The method for producing marine biodegradable polymer particles of 10 above wherein, in the crosslinking step, the crosslinking reaction is carried out by independently adding at least one of the two or more salt compounds.

12. An ultraviolet scattering agent containing the marine biodegradable polymer particles of any of 1 to 9 above.

13. A marine biodegradable additive containing the marine biodegradable polymer particles of any of 1 to 9 above.

14. A personal care product containing the marine biodegradable additive of 13 above.

15. A cosmetic containing the marine biodegradable additive of 13 above.

16. A coating containing the marine biodegradable additive of 13 above.

17. An ink containing the marine biodegradable additive of 13 above.

18. A resin composition containing the marine biodegradable additive of 13 above.

19. A formed body containing the marine biodegradable additive of 13 above.

## ADVANTAGEOUS EFFECTS OF INVENTION

The marine biodegradable polymer particles of the invention, by using together two or more types of at least divalent metal cations, stabilizes the structure and at the same time, compared with an egg-box crosslinked structure due to crosslinking by a single type of metal cation, moderately destabilizes the structure and adjusts the crosslink density, thereby having an unprecedented set of qualities, including suitable softness, smoothness, flexibility, the feel of metal, and optical properties such as UV scattering properties. Moreover, the biodegradation within the ocean of compositions and formed bodies containing the marine biodegradable polymer particles of the invention is accelerated, making these particles useful as a measure against marine pollution. The marine biodegradable polymer particles of the invention are thus useful as a substitute for existing polymer particles utilized in various applications.

## DESCRIPTION OF EMBODIMENTS

[Marine Biodegradable Polymer Particles]

The marine biodegradable polymer particles of the invention are composed of a polymer compound in which water-soluble polymeric polyvalent anions having monovalent anionic substituents are crosslinked through two or more types of at least divalent metal cations, the water-soluble polymeric polyvalent anions including at least one anion derived from alginic acid, and satisfy conditions (1) to (4) below:

(1) the content of the at least divalent metal cations included in the particles is from 3 to 30 wt%;
(2) the difference between the largest and smallest atomic radii of the metal elements making up the at least divalent metal cations is 15 Å or more;
(3) the content of the at least divalent metal cations derived from metal elements having an atomic radius of 150 Å or more is at least 25 wt% of all the at least divalent metal cations; and
(4) the particles have a water absorption of less than 300 mL/100 g.

Examples of the monovalent anionic substituent include the carboxylate anion ($-COO^-$), the sulfonate anion ($-SO_3^-$), the sulfate anion ($-O-SO_3^-$) and the phosphate anion ($-P(=O)(OH)-O^-$). Of these, the carboxylate anion, the sulfonate anion and the sulfate anion are preferred. From an environmental standpoint in particular, the carboxylate anion is preferred.

The starting material for the water-soluble anionic polymer is of biomass origin; one derived from a natural polymer is best because it has a biodegradable structure. The water-soluble anionic polymer is preferably one derived from a polysaccharide.

The water-soluble anionic polymer is preferably, for example, a monovalent salt of a polysaccharide. Specific examples include monovalent salts of alginic acid, such as sodium alginate, potassium alginate and ammonium alginate; monovalent salts of carboxymethylcellulose (CMC) and monovalent salts of cellulose derivatives, such as sodium CMC, potassium CMC and ammonium CMC; monovalent salts of modified starches and monovalent salts of starch derivatives, such as starch sodium octenyl succinate and sodium starch glycolate; monovalent salts of glycosaminoglycan derivatives, including monovalent salts of hyaluronic acid such as sodium hyaluronate and monovalent salts of chondroitin sulfate such as sodium chondroitin sulfate; monovalent salts of chitosan derivatives; monovalent salts of chitin derivatives; monovalent salts of carrageenan such as sodium carrageenan, and monovalent salts of carrageenan derivatives; monovalent salts of pectic acid such as sodium polypectate, and monovalent salts of pectin derivatives; monovalent salts having natural gum (e.g., gum arabic, xanthan gum, gellan gum, gum tragacanth, guar gum) backbones, and monovalent salts of derivatives thereof; and monovalent salts having agar backbones, as well as monovalent salts of derivatives thereof. Of these, the use of a monovalent salt of alginic acid alone as the water-soluble anionic polymer, or the use of a monovalent salt of alginic acid in combination with at least one type of compound selected from monovalent salts of CMC, monovalent salts of cellulose derivatives, monovalent salts of starch octenyl succinate, monovalent salts of starch derivatives, monovalent salts of hyaluronic acid and monovalent salts of chondroitin sulfate is preferred.

The viscosity of a 1 wt% or 10 wt% aqueous solution of the water-soluble anionic polymer is preferably from 0.01 to 2,000 mPa·s, more preferably from 0.1 to 1,000 mPa·s, and most preferably from 1.0 to 500 mPa·s. Taking into consideration the productivity, it is more preferable for the viscosity of the 10 wt% aqueous solution to satisfy the above range. This viscosity is the measured value at 20°C obtained with a Brookfield viscometer (model BL).

Examples of the at least divalent metal cations include, without particular limitation, calcium, strontium, beryllium, magnesium, aluminum, zinc, iron, copper, barium, platinum, gold, radium, nickel, cobalt and manganese ions. Of these, beryllium, magnesium, calcium, strontium, barium, zinc and aluminum ions are preferred. Based on solubility, general utility and environmental considerations, calcium, strontium, magnesium, aluminum and zinc ions are preferred.

The content (total weight) of the at least divalent metal cations included in the marine biodegradable polymer particles is preferably from 3 to 30 wt%, more preferably from 4 to 25 wt%, even more preferably from 5 to 20 wt%, and most preferably from 6 to 18 wt%. At a content of at least divalent metal cations within the above range, major deformation due to water does not occur and the particle characteristics can be maintained.

The sizes of the atoms of the metal elements that provide the above metal cations are important in adjusting the crosslink density. Because materials which use naturally occurring ingredients such as polysaccharides have a structure that has a good affinity to water and readily dissolves or swells, to obtain a suitable softness and texture while at the same time maintaining a structure that does not dissolve or swell in water, it is necessary to consider the sizes and compounding ratios of the atoms and adjust the crosslink density so as to strike a good balance.

As a result of investigations by the inventors, a suitable softness and texture were found to be achievable with the combined use of atoms for which the difference between the largest atomic radius and the smallest atomic radius is 15 Å or more. It was also confirmed that optical properties such as diffusivity dramatically improve with the combined use of a plurality of divalent metal cations.

The lower limit in the above difference in atomic radii is 15 Å, preferably 20 Å, more preferably 25 Å, and still more preferably 30 Å. There is no particular upper limit in this difference, so long as the crosslink density can be adjusted. However, when the crosslink density is too small (few crosslink sites), a structure that is prone to dissolution or swelling results and adjusting the crosslink density becomes difficult. Therefore, the practical upper limit is preferably 100 Å, more preferably 90 Å, and even more preferably 80 Å. In this invention, the atomic radii are the numerical values given in the Diploma Program (DP) Kagaku Shiryo-shu (the Japanese language edition, published in August 2015 and revised in May 2016, of the original English-language Chemistry data booklet published in June 2014).

It is essential for the metal cations to include at least one type of metal cation from metal elements having atomic radii of 150 Å or more and for the content of such metal cations to be at least 25 wt% of all at least divalent metal cations. In this case, marine biodegradable polymer particles of improved tactile feel such as softness and texture and good optical characteristics such as diffusivity are obtained. The content of metal cations from metal elements having atomic radii of 150 Å or more is preferably at least 30 wt%, more preferably at least 35 wt%, even more preferably at least 40 wt%, and most preferably at least 50 wt%. On the other hand, the upper limit of this content is 100 wt% or less, although in cases where the subsequently described metal cations from metal elements having atomic radii of less than 150 Å are included, the upper limit is preferably 95 wt% or less, and more preferably 90 wt% or less. The content of metal cations is a value measured by inductively coupled plasma mass spectrometry (ICP-MS).

In cases where the above metal cations include two or more types from metal elements having atomic radii of 150 Å or more, the content ratio therebetween is not particularly limited, although the content ratio by weight of the metal cations having a small atomic radius to the metal cations having a large atomic radius is preferably from 1:99 to 99: 1, and more preferably from 10:90 to 90:10.

In addition to metal cations from metal elements having an atomic radius of 150 Å or more, the metal cations may also include metal cations from metal elements having an atomic radius of less than 150 Å. Some metal elements with an atomic radius of less than 150 Å have functionality and can impart such qualities as antimicrobial properties, antifungal properties, deodorizing properties and UV-blocking properties. In particular, by including metal cations from metal elements having atomic radii of less than 150 Å, the UV scattering effects are greatly enhanced. In such cases, the marine biodegradable polymer particles of the invention are also useful as a UV scattering agent. In cases where two or more types of metal cations from metal elements having atomic radii of less than 150 Å are included, the content ratio of these is not particularly limited, although the content ratio by weight of the metal cations having a small atomic radius to the metal cations having a large atomic radius is preferably from 1:99 to 99:1, more preferably from 10:90 to 90:10, and most preferably from 20:80 to 80:20.

The atomic radii of the metal elements that form the at least divalent metal cations are preferably from 80 to 220 Å, more preferably from 90 to 210 Å, and even more preferably from 100 to 200 Å.

Given the above, from the standpoints of the balance in crosslinkability, improving tactile feel such as softness and texture, and optical characteristics such as diffusivity, it is preferable for the at least divalent metal cations to include calcium ions and/or strontium ions as the primary constituents. In particular, it is preferable to include calcium ions or strontium ions, and at least one type of metal cation selected from among magnesium, barium, zinc and aluminum ions.

Because the marine biodegradable polymer particles of the invention are targeted at useful applications where they maintain their shape in ordinary use, swelling and excessive water absorption in ordinary water (tap water, ionized water, purified water, etc.) must be avoided. Hence, the water absorption of the marine biodegradable polymer particles of the invention is preferably less than 300 mL/100 g, more preferably less than 250 mL/100 g, and even more preferably less than 200 mL/100 g. When the subsequently described hydrophobizing treatment has been carried out, the water absorption is preferably less than 150 mL/100 g, and more preferably less than 100 mL/100 g.

The marine biodegradable polymer particles of the invention must have, in applications where softness and feel are regarded as important, characteristics like those of existing polymer particles. One such particle characteristic that can be evaluated is the compressive strength of a single particle when deformed to a displacement of X% of the particle diameter, which property is indicated here as the X% compressive strength. For example, the 10% compressive strength $K_{10}$ at 10% displacement of the particle diameter is the value determined from the following formula after measurement of the load value (test strength) based on the particle diameter and the compressive displacement using a micro-compression tester (MCT-W201, from Shimadzu Corporation).

$$10\% \text{ compressive strength } K_{10} = 2.48 \times P_{10}/(\pi d^2)$$

$P_{10}$: load (N) at 10% displacement of particle diameter
$\pi$: circular constant
d: particle diameter (mm)

The compressive strength properties when the particle diameter has been displaced universally and quantitatively express the strength of the particle. By using the 10% compressive strength $K_{10}$ obtained by a compression test as in this invention, it is possible to quantitatively and unambiguously express the suitable softness of the particle.

In the case of ordinary polymer particles, the 10% compressive strength $K_{10}$ is from about 0.1 MPa to about 5,000 MPa. However, the marine biodegradable polymer particles of the invention, in applications where softness and feel are regarded as important, have a 10% compressive strength $K_{10}$ that is preferably from 1 to 1,000 MPa, more preferably from 10 to 500 MPa, even more preferably from 15 to 300 MPa, and most preferably from 20 to 200 MPa. In applications where even further softness is desired, it is possible to adjust the 10% compressive strength $K_{10}$ within the range of 20 to 150 MPa, or even within the range of 20 to 100 MPa, by suitably adjusting the types and content ratios of the at least divalent metal ions.

The marine biodegradable polymer particles of the invention preferably have heat resistance. Specifically, it is preferable for the melting temperature to be at least 150°C. The melting temperature is preferably at least 160°C, more preferably at least 180°C, and even more preferably at least 200°C. The melting temperature can be adjusted by adjusting the degree of crosslinking. It is also possible, by way of the degree of crosslinking, to have the particles decompose without melting.

The marine biodegradable polymer particles of the invention may include from 0.01 to 10 wt% of monovalent cations. Examples of such monovalent cations include monovalent metal cations such as the lithium ion, sodium ion, potassium ion and silver ion; and monovalent organic ions such as the ammonium cation. Of these, metal cations are preferred; the sodium ion and the potassium ion are more preferred. By having monovalent cations present within the above range, the crosslink density decreases, which appears to serve as one factor that imparts properties such as softness and flexibility.

To achieve a good degree of crosslinking, it is preferable for the at least divalent metal cations in the marine biodegradable polymer particles of the invention to have an equivalent weight of from 180 g/eq to 1,000 g/eq. The ratio of the equivalent weight of the at least divalent metal cations to the equivalent weight of the anionic substituents on the polymeric polyvalent anions is preferably from 0.2:1 to 0.5:1.

The marine biodegradable polymer particles of the invention have an average particle size which is preferably 5 mm or less, and more preferably, with increasing desirability in the following order: 1 mm or less, 500 μm or less, 100 μm or less, 60 μm or less, 30 μm or less, 15 μm or less, 10 μm or less. The lower limit is preferably 0.1 μm, more preferably 0.5 μm, and even more preferably 1.0 μm. In this invention, the average particle size refers to the volume mean particle diameter (MV) obtained by the laser diffraction scattering method.

The marine biodegradable polymer particles of the invention have a shape which, although not particularly limited, may be physically or chemically shape-controlled, or may be a physically pulverized shape, such as a spherical, approximately spherical, flattened or recessed shape. From the standpoint of the texture, slip characteristics and control of the particle size distribution, particles that are physically or chemically shape-controlled, such as spherical, approximately spherical, elliptical, flattened or recessed particles, are preferred. Particles that are formed of curves without sharp edges, such as approximately spherical, elliptical, flattened or recessed shapes, have good optical properties and therefore are more preferred.

In addition, when the marine biodegradable polymer particles of the invention are dispersed in a 3 wt% aqueous solution of sodium chloride to a particle concentration of 0.1 wt%, the phenomenon of increasing transparency as the shapes of the particles change and dissolution occurs can be confirmed in 72 hours or 240 hours. When the phenomenon indicated by dissolution, shape change, etc. cannot be confirmed in at least about 240 hours, environmental pollution in the oceans (marine contamination), the adsorption of chemical substances and adverse impacts on ecosystems become a concern, and so retention of the particle shapes for such an excessively long period of time is undesirable.

Where necessary, a hydrophobizing agent may be used to carry out hydrophobizing treatment on the marine biodegradable polymer particles of the invention.

The compound used in such hydrophobizing treatment (which compound is also referred to below as the "hydrophobizing agent") is a compound having the function of imparting the marine biodegradable polymer particles with hydrophobicity or water-repellency, and may be suitably selected provided that it is a compound which does not have adverse effects on the marine biodegradability of the resulting polymer particles overall, on environmental contamination and on the ecosystem.

The hydrophobizing agent is preferably a salt compound having an anion of 6 or more carbon atoms. The number of carbon atoms on the anion is more preferably 10 or more, and even more preferably 12 or more. Although there is no upper limit in the number of carbon atoms on the anion, 30 or fewer are preferred, 25 or fewer are more preferred, and 20 or fewer are even more preferred. The salt compound is preferably a salt compound having a monovalent cation, and more preferably a salt compound having a monovalent metal cation. The salt compound is preferably one which dissolves in water at room temperature or up to 80°C. Such a compound is readily compatible in seawater, enabling a good rate of biodegradation to be achieved. Also, a hydrophobizing agent which includes, as a hydrophilic group, at least one entity selected from the carboxyl group, the ester bond, the sulfone group, the ether bond and the amide bond is preferred.

Specific examples of the hydrophobizing agent include carboxylic acid salts, salts of amino acid derivatives, sulfate ester salts, sulfonic acid salts, phosphate ester salts and lactate ester salts. The hydrophobizing agent, when designed out of environmental considerations so as to fully satisfy the solubility in water and saltwater and the microbial degradability in the environment, is preferably one having a molecular weight of 5,000 or less, more preferably one having a molecular weight of from 50 to 1,000, even more preferably one having a molecular weight of from 100 to 600, and most preferably one having a molecular weight of from 200 to 500.

The above carboxylic acid salts are preferably salts of carboxylic acids having from 6 to 30 carbon atoms, more preferably salts of carboxylic acids having from 10 to 25 carbon atoms, and even more preferably salts of carboxylic acids having from 12 to 20 carbon atoms. The carboxylic acid salts may be salts of monocarboxylic acids or salts of polycarboxylic acids.

Examples of the monocarboxylic acids include caproic acid, enanthic acid, caprylic acid, octanoic acid, pelargonic acid, capric acid, undecylenic acid, lauric acid, myristic acid, pentadecanoic acid, palmitic acid, palmitoleic acid, margaric acid, stearic acid, isostearic acid, oleic acid, vaccenic acid, ricinolic acid, linoleic acid, linolenic acid, eleostearic acid, oxystearic acid, arachidic acid, mead acid, arachidonic acid, eicosapentaenoic acid, behenic acid, docosahexaenoic acid, lignoceric acid, nervonic acid, cerotic acid, montanic acid, melissic acid, coconut oil fatty acid and palm oil fatty acid. Isomers of these having branched structures are also acceptable.

Examples of the polycarboxylic acids include octanedioic acid (suberic acid), nonanedioic acid (azelaic acid), decanedioic acid (sebacic acid), undecanedioic acid, dodecanedioic acid, tridecanedioic acid, tetradecanedioic acid, pentadecanedioic acid, hexadecanedioic acid, heptadecanedioic acid, octadecanedioic acid, nonadecanedioic acid and eicosanedioic acid. Isomers of these having a branched structure and polycarboxylic acids with a functionality of three or more may also be used.

The monocarboxylic acid salts are preferably monovalent metal salts, specific examples of which include caprylic acid salts such as potassium caprylate and sodium caprylate; octanoic acid salts such as potassium octanoate and sodium octanoate; pelargonic acid salts such as potassium pelargonate and sodium pelargonate; capric acid salts such as potassium caprate and sodium caprate; undecylenic acid salts such as potassium undecylenate and sodium undecylenate; lauric acid salts such as potassium laurate and sodium laurate; myristic acid salts such as potassium myristate and sodium myristate; pentadecanoic acid salts such as potassium pentadecanoate and sodium pentadecanoate; palmitic acid salts such as potassium palmitate and sodium palmitate; margaric acid salts such as potassium margarate and sodium margarate; stearic acid salts such as potassium stearate and sodium stearate; isostearic acid salts such as potassium isostearate and sodium isostearate; oleic acid salts such as potassium oleate and sodium oleate; linoleic acid salts such as potassium linoleate and sodium linoleate; linolenic acid salts such as potassium linolenate and sodium linolenate; arachidonic acid salts such as potassium arachidonate and sodium arachidonate; behenic acid salts such as potassium behenate and sodium behenate; docosahexaenoic acid salts such as sodium docosahexanoate; and coconut fatty acid salts such as potassium cocoate and sodium cocoate. Of these, salts of carboxylic acids having from 10 to 20 carbon atoms, such as lauric acid salts, myristic acid salts, stearic acid salts and arachidonic acid salts, are preferred.

The polycarboxylic acid salts are preferably monovalent metal salts, specific examples of which include octanedioic acid salts such as disodium octanedioate and dipotassium octanedioate; nonanedioic acid salts such as disodium nonanedioate and dipotassium nonanedioate; decanedioic acid salts such as disodium decanedioate and dipotassium decanedioate; undecanedioic acid salts such as disodium undecanedioate and dipotassium undecanedioate; dodecanedioic acid salts such as disodium dodecanedioate and dipotassium dodecanedioate; tridecanedioic acid salts such as disodium tridecanedioate and dipotassium tridecanedioate; tetradecanedioic acid salts such as disodium tetradecanedioate and dipotassium tetradecanedioate; pentadecanedioic acid salts such as disodium pentadecanedioate and dipotassium pentadecanedioate; hexadecanedioic acid salts such as disodium hexadecanedioate and dipotassium hexadecanedioate; heptadecanedioic acid salts such as disodium heptadecanedioate and dipotassium heptadecanedioate; octadecanedioic acid salts such as disodium octadecanedioate and dipotassium octadecanedioate; nonadecanedioic acid salts such as disodium nonadecanedioate and dipotassium nonadecanedioate; and eicosanedioic acid salts such as disodium eicosanedioate and dipotassium eicosanedioate. Isomers of these having a branched structure and salts of polycarboxylic acids with a functionality of three or more may also be used. Of these, salts of dicarboxylic acids having from 10 to 20 carbon atoms, such as decanedioic acid salts, dodecanedioic acid salts, tetradecanedioic acid salts, hexadecanedioic acid salts, octadecanedioic acid salts and eicosanedioic acid salts are preferred.

The salts of amino acid derivatives are preferably ones having from 6 to 30 carbon atoms, more preferably ones having from 10 to 25 carbon atoms, and even more preferably ones having from 12 to 20 carbon atoms. The salts of amino acid derivatives are preferably monovalent salts, and more preferably monovalent metal salts.

Examples of the amino acid derivatives include sarcosine derivatives such as capryloyl sarcosine, lauroyl sarcosine, myristoyl sarcosine, palmitoyl sarcosine and cocoyl sarcosine; glutamic acid derivatives such as capryloyl glutamic acid, lauroyl glutamic acid, myristoyl glutamic acid, palmitoyl glutamic acid, stearoyl glutamic acid, cocoyl acyl glutamic acid, cocoyl glutamic acid, acyl glutamic acid and dilauroyl glutamic acid; glycine derivatives such as lauroyl glycine, myristoyl glycine, palmitoyl glycine, palmitoyl methylglycine, cocoyl acyl glycine and cocoyl glycine; alanine derivatives such as lauroyl methylalanine, myristoyl methylalanine, cocoyl alanine and cocoyl methylalanine; lysine derivatives such as lauroyl lysine, myristoyl lysine, palmitoyl lysine, stearoyl lysine, oleyl lysine and acylated lysine; aspartic acid derivatives such as lauroyl aspartic acid, myristoyl aspartic acid, palmitoyl aspartic acid and stearoyl aspartic acid; taurine derivatives such as lauroyl taurine, lauroyl methyltaurine, myristoyl taurine, myristoyl methyltaurine, palmitoyl taurine, palmitoyl methyltaurine, stearoyl taurine and stearoyl methyltaurine; and proline derivatives such as lauroyl proline, myristoyl proline and palmitoyl proline. N-acyl derivatives of amino acids are especially preferred.

The salts of amino acid derivatives are exemplified by the salts of amino acid derivatives having hydrocarbon groups, including salts of sarcosine derivatives, such as potassium capryloyl sarcosinate, sodium capryloyl sarcosinate, potassium lauroyl sarcosinate, sodium lauroyl sarcosinate, potassium myristoyl sarcosinate, sodium myristoyl sarcosinate, potassium palmitoyl sarcosinate, sodium palmitoyl sarcosinate, potassium cocoyl sarcosinate and sodium cocoyl sarcosinate; salts of glutamic acid derivatives, such as potassium capryloyl glutamate, sodium capryloyl glutamate, potassium lauroyl glutamate, sodium lauroyl glutamate, potassium myristoyl glutamate, sodium myristoyl glutamate, sodium palmitoyl glutamate, magnesium palmitoyl glutamate, potassium stearoyl glutamate, sodium stearoyl glutamate, potassium cocoyl acyl glutamate, sodium cocoyl acyl glutamate, potassium cocoyl glutamate, sodium cocoyl glutamate, potassium acyl glutamate, sodium acyl glutamate, sodium dilauroyl glutamate lysine and sodium polyglutamate; salts of glycine derivatives, such as potassium lauroyl glycine, sodium lauroyl glycine, potassium myristoyl glycine, sodium myristoyl glycine, sodium palmitoyl glycine, sodium palmitoyl methylglycine, potassium cocoyl acyl glycine, sodium cocoyl acyl glycine, potassium cocoyl glycine and sodium cocoyl glycine; salts of alanine derivatives, such as potassium lauroyl methylalanine, sodium lauroyl methylalanine, sodium myristoyl methylalanine, sodium cocoyl alanine and sodium cocoyl methylalanine; salts of aspartic acid derivatives, such as potassium lauroyl aspartate, sodium lauroyl aspartate, potassium myristoyl aspartate, sodium myristoyl aspartate, potassium palmitoyl aspartate, sodium palmitoyl aspartate, potassium stearoyl aspartate and sodium stearoyl aspartate; salts of taurine derivatives, such as sodium lauroyl taurate, potassium lauroyl taurate, sodium lauroyl methyltaurate, potassium myristoyl taurate, sodium myristoyl taurate, sodium myristoyl methyltaurate, potassium palmitoyl taurate, sodium palmitoyl taurate, potassium palmitoyl methyltaurate, sodium palmitoyl methyltaurate, potassium stearoyl taurate, sodium stearoyl taurate and sodium stearoyl methyltaurate; and salts of proline derivatives, such as sodium lauroyl proline, sodium myristoyl proline and sodium palmitoyl proline. N-acyl derivative salts of amino acids are especially preferred.

Examples of sulfate ester salts include alkyl sulfate ester salts, polyoxyethylene aryl ether sulfate ester salts, polyoxyethylene alkyl ether sulfate ester salts, polyoxyalkylene alkyl ether sulfate ester salts, polyoxyalkylene alkenyl ether sulfate salts and polyoxyethylene castor oil ether sulfate ester salts. The sulfate ester salts are preferably monovalent salts; ammonium salts and monovalent metal salts are more preferred.

Specifically, the alkyl sulfate ester salt is preferably one having an alkyl group of 6 to 30 carbon atoms, more preferably one have an alkyl group of 10 to 25 carbon atoms, and even more preferably one having an alkyl group of 12 to 20 carbon atoms. Specific examples include potassium lauryl sulfate, sodium lauryl sulfate, ammonium lauryl sulfate, potassium myristyl sulfate, sodium myristyl sulfate, ammonium myristyl sulfate, sodium cetyl sulfate, ammonium cetyl sulfate, sodium stearyl sulfate, ammonium stearyl sulfate, sodium oleyl sulfate and ammonium oleyl sulfate.

The polyoxyethylene aryl ether sulfate ester salts are preferably ones having a hydrophilic-lipophilic balance (HLB) of 16 or less, and more preferably ones having an HLB of 12 or less. Specific examples include polyoxyethylene polycyclic phenyl ether sulfate ester salts such as sodium polyoxyethylene polycyclic phenyl ether sulfate esters and ammonium polyoxyethylene polycyclic phenyl ether sulfate esters; and sodium polyoxyethylene aryl ether sulfate esters.

The polyoxyethylene alkyl ether sulfate ester salts are preferably ones having an HLB of 16 or less, and more preferably ones having an HLB of 12 or less. Specific examples include sodium polyoxyethylene lauryl ether sulfate, ammonium polyoxyethylene lauryl ether sulfate, sodium polyoxyethylene myristyl ether sulfate, ammonium polyoxyethylene myristyl ether sulfate, sodium polyoxyethylene cetyl ether sulfate, ammonium polyoxyethylene cetyl ether sulfate, sodium polyoxyethylene stearyl ether sulfate, ammonium polyoxyethylene stearyl ether sulfate, sodium polyoxyethylene oleyl ether sulfate and ammonium polyoxyethylene oleyl ether sulfate.

The polyoxyalkylene alkyl ether sulfate ester salts are preferably ones having an HLB of 16 or less, and more preferably ones having an HLB of 12 or less. Specific examples include sulfate ester sodium salts of polyoxyethylene-polyoxypropylene block copolymers, sulfate ester sodium salts of polyoxyethylene-polyoxybutylene block copolymers, and sulfate ester sodium salts of alkyl ethers of polyoxyethylene-polyoxypropylene block copolymers. The polyoxyalkylene alkenyl ether sulfate salts are preferably ones having an HLB of 16 or less, and more preferably ones having an HLB of 12 or less. Specific examples include sulfate ester ammonium salts of alkenyl ethers of polyoxyethylene-polyoxyalkylene block copolymers. The polyoxyethylene castor oil ether sulfate esters and salts thereof are preferably ones having an HLB of 16 or less, and more preferably ones having an HLB of 12 or less. Specific examples include polyoxyethylene castor oil ether sulfate esters and ammonium salts of polyoxyethylene castor oil ether sulfate esters.

The sulfonic acid salts are preferably ones having from 6 to 30 carbon atoms, more preferably ones having from 10 to 25 carbon atoms, and even more preferably ones having from 12 to 20 carbon atoms. The sulfonic acid salts are preferably monovalent salts, and more preferably ammonium salts or monovalent metal salts. Specific examples include alkyl sulfonic acid salts such as sodium lauryl sulfonate, ammonium lauryl sulfonate, sodium myristyl sulfonate, ammonium myristyl sulfonate, sodium cetyl sulfonate, ammonium cetyl sulfonate, sodium stearyl sulfonate, ammonium stearyl sulfonate, sodium oleyl sulfonate and ammonium oleyl sulfonate; dodecylbenzene sulfonic acid salts such as ammonium dodecylbenzene sulfonate and sodium dodecylbenzene sulfonate; alkylene disulfonic acid salts sodium alkylene disulfonates; dialkyl succinate sulfonic acid salts such as sodium dialkyl succinate sulfonates; monoalkyl succinate sulfonic acid salts such as disodium monoalkyl succinate sulfonates; naphthalene sulfonic acid formalin condensate salts, such as sodium salts of naphthalene sulfonic acid formalin condensates; olefin sulfonic acid salts such as sodium olefin sulfonates and ammonium olefin sulfonates; isethionic acid salts such as potassium lauroyl isethionate, sodium lauroyl isethionate, sodium myristoyl isethionate, sodium palmitoyl isethionate and sodium stearoyl isethionate; and sulfosuccinic acid salts such as sodium dihexyl sulfosuccinate, sodium dioctyl sulfosuccinate, ammonium dioctyl sulfosuccinate, sodium didecyl sulfosuccinate and sodium diisobutyl sulfosuccinate. Of these, sulfonic acid salts having an alkyl group of 12 to 20 carbon atoms are especially preferred.

The phosphate ester salts are exemplified by alkyl phosphate ester salts. The alkyl phosphate ester salts are preferably ones having an alkyl group of 6 to 30 carbon atoms, more preferably ones having an alkyl group of 10 to 25 carbon atoms, and even more preferably ones having an alkyl group of 12 to 20 carbon atoms. Specific examples include octyl phosphate salts such as potassium octyl phosphate; nonyl phosphate salts such as potassium nonyl phosphate; decyl phosphate salts such as potassium decyl phosphate; undecyl phosphate salts such as potassium undecyl phosphate; lauryl phosphate salts such as potassium lauryl phosphate; myristyl phosphate salts such as potassium myristyl phosphate; cetyl phosphate salts such as potassium cetyl phosphate and sodium cetyl phosphate; and stearyl phosphate salts such as potassium stearyl phosphate.

The lactate ester salts are preferably ones having from 6 to 30 carbon atoms, more preferably ones having from 10 to 25 carbon atoms, and even more preferably ones having from 12 to 20 carbon atoms. Specific examples include sodium stearoyl lactate, potassium stearoyl lactate, sodium isostearoyl lactate and sodium lauroyl lactate.

Of these, carboxylic acid salts, salts of amino acid derivatives, sulfate ester salts and sulfonic acid salts are preferred as the hydrophobizing agent. In particular, salts having an organic ion as the cation and salts having a metal cation are preferred from an environmental standpoint. The organic ion is preferably a monovalent organic ion such as an ammonium ion. The metal cation is preferably a monovalent metal cation such as a lithium ion, potassium ion, sodium ion or silver ion. Of these, from environmental, biological safety, versatility, cost and other standpoints, sodium, potassium and ammonium ions are preferred, sodium and potassium ions are more preferred, and sodium ions are even more preferred.

With the use of such hydrophobizing agents, anions on the hydrophobizing agent attach by way of ionic bonds to some of the divalent metal cations included on the crosslinked polymer and the resulting crosslinked polymer exhibits hydrophobicity.

Additives such as carboxylic acids, amino acid derivatives, organosilicon compounds, silicone compounds, fluorine compounds, sulfate esters, sulfonic acids, phosphate esters, lactic acid esters, oils, acrylic compounds, acrylic resins, titanium coupling agents, inorganic compounds, metal oxides, solid lubricants and surfactants may be added to the hydrophobizing agent, insofar as doing so does not compromise the advantageous effects of the invention.

The carboxylic acids may be monocarboxylic acids or polycarboxylic acids; specific examples include those mentioned earlier. Specific examples of the amino acid derivatives include those mentioned earlier.

The organosilicon compounds are exemplified by alkylated silanes having at least one alkyl group of 6 to 30 carbon atoms, alkylated silazanes having at least one alkyl group of 6 to 30 carbon atoms, trialkoxysilanes have at least one alkoxy group of 6 to 30 carbon atoms, octyltrialkoxysilanes, triethoxycaprylylsilane and 3-methacryloxypropyltrimethoxysilane.

The silicone compounds are exemplified by methylhydrogenpolysiloxane, methylpolysiloxane (methicone), dimethylpolysiloxane (dimethicone), triethoxysilylethyl polydimethylsiloxyethyl dimethicone, methylphenylpolysiloxane, triethoxysilylethyl polydimethylsiloxyethyl hexyl dimethicone, cyclic silicones, crosslinked silicones, acrylic-silicone graft polymers, organosilicone resins, partially crosslinked organopolysiloxane polymers, tetramethyltetrahydrogencyclotetrasiloxane, trimethylsiloxysilicic acid, amino-modified silicones, carboxylic acid-modified silicones, fluorinated silicones, silicone gums, acrylic silicones, silicone resins, triethoxysilylethyl polydimethylsiloxyethyl dimethicone, triethoxysilylethyl polydimethylsiloxyethyl hexyl dimethicone and fluorinated silicones.

Examples of the fluorine compounds include perfluoroalkylphosphate esters, perfluoroalkylsilanes, perfluoroalkylalkoxysilanes, perfluoroalkyl group-containing esters, salts of perfluoroalkylphosphate esters, perfluoropolyethers, fluorosilicones, fluorinated silicone resins, trimethoxy(3,3,3-trifluoropropyl)silane and tridecafluorooctyltriethoxysilane.

Examples of sulfate esters include alkyl sulfate esters, polyoxyethylene aryl ether sulfate esters, polyoxyethylene alkyl ether sulfate esters and polyoxyethylene castor oil ether sulfate esters. The alkyl sulfate esters are preferably ones having an alkyl group of 6 to 30 carbon atoms, specific examples of which include lauryl sulfate, myristyl sulfate, cetyl sulfate, stearyl sulfate and oleyl sulfate. The polyoxyethylene aryl ether sulfate esters are preferably ones having an HLB of 16 or less, and more preferably ones having an HLB of 12 or less. Specific examples include polyoxyethylene polycyclic phenyl ether sulfate esters and polyoxyethylene aryl ether sulfate esters. The polyoxyethylene alkyl ether sulfate esters are preferably ones having an HLB of 16 or less, and more preferably ones having an HLB of 12 or less.

The sulfonic acids are preferably ones having from 6 to 30 carbon atoms. Specific examples include alkyl sulfonic acids such as lauryl sulfonic acid, myristyl sulfonic acid, cetyl sulfonic acid, stearyl sulfonic acid and oleyl sulfonic acid; dodecylbenzene sulfonic acid; alkylene disulfonic acids; dialkylsuccinate sulfonic acids; monoalkylsuccinate sulfonic acids; naphthalene sulfonic acid-formalin condensates; olefin sulfonic acids; isethionic acids such as lauroyl isethionic acid, myristoyl isethionic acid, palmitoyl isethionic acid and stearoyl isethionic acid; and sulfosuccinic acid.

The phosphate esters are exemplified by polyoxyalkylene alkyl ether phosphate esters and alkyl phosphate esters. The polyoxyalkylene alkyl ether phosphate esters are preferably ones having an HLB of 16 or less, and more preferably ones having an HLB of 12 or less. Specific examples include polyoxyethylene (2) stearyl ether phosphate esters.

The alkyl phosphate esters are preferably ones having an alkyl group of 6 to 30 carbon atoms. Specific examples include octyl phosphoric acid, nonyl phosphoric acid, decyl phosphoric acid, undecyl phosphoric acid, lauryl phosphoric acid, myristyl phosphoric acid, cetyl phosphoric acid and stearyl phosphoric acid.

The lactic acid esters are preferably ones having from 6 to 30 carbon atoms. Specific examples include lauryl lactate, myristyl lactate, cetyl lactate, oleyl lactate and octyldodecyl lactate.

Examples of the oils include petrolatum, liquid paraffins, squalane, paraffin wax, linseed oil, cottonseed oil, coconut oil, castor oil, egg oil, lanolin fatty acids, propylene glycol dicaprate, glyceryl trioctanoate, cetyl 2-ethylhexanoate, isocetyl stearate, stearyl alcohol, cetyl alcohol, oleyl alcohol, beef tallow, beeswax, spermaceti, Japan wax, lanolin, carnauba wax and candelilla wax.

Examples of the acrylic compounds include alkyl (meth)acrylates. Examples of the acrylic resins include copolymers of (meth)acrylic acid and styrene compounds, as well as salts thereof; copolymers of (meth)acrylic acid and (meth)acrylate ester compounds, as well as salts thereof; copolymers of (meth)acrylic acid and vinyl ester compounds, as well as salts thereof; copolymers of (meth)acrylic acid and olefin compounds, as well as salts thereof; and copolymers of (meth)acrylic acid and conjugated diene compounds, as well as salts thereof.

Examples of the titanium coupling agents include alkyl titanates, pyrophosphoric acid titanates, phosphonic acid titanates and amino acid titanates.

An example of the inorganic compounds is alumina. An example of the metal oxides is titanium oxide.

Examples of the solid lubricants include polyolefin waxes (e.g., polyethylene wax), paraffin waxes (e.g., synthetic paraffins, natural paraffins), fluoropolymer waxes (e.g., polytetrafluoroethylene), fatty amide compounds (e.g., stearamide, palmitamide), metal sulfides (e.g., molybdenum disulfide, tungsten disulfide), graphite, graphite fluoride, boron nitride, polyalkylene glycols and alkali metal sulfates.

The hydrophobizing treatment method will be described later in detail.

[Method for Producing Marine Biodegradable Polymer Particles]

The method for producing the marine biodegradable polymer particles of the invention includes the step of crosslinking water-soluble polymeric polyvalent anions derived from a water-soluble anionic polymer containing at least a monovalent salt of alginic acid and having monovalent anionic substituents within:

(A) a medium in which particles of the water-soluble anionic polymer have been dispersed to a concentration of at least 5 wt%,
(B) a medium in which, using water and an oily medium, the water-soluble anionic polymer has been suspended or emulsified in water to a concentration of at least 5 wt%, or
(C) a medium in which the water-soluble anionic polymer has been hydrophilized or dissolved to a concentration of at least 5 wt%

by using as crosslinking agents two or more salt compounds containing at least divalent metal cations.

The crosslinking reaction is carried out by adding the two or more salt compounds a plurality of times in divided portions such that the difference between the largest and smallest atomic radii of the metal elements making up the at least divalent metal cations included in the salt compound is 15 Å or more, and the content of at least divalent metal cations derived from metal elements having an atomic radius of 150 Å or more becomes at least 25 wt% of all the at least divalent metal cations.

The method which uses the medium in (A) is referred to below as Method 1, the method which uses the medium in (B) is referred to as Method 2, and the method which uses the medium in (C) is referred to as Method 3.

Method 1 is a method which includes the step of crosslinking water-soluble polymeric polyvalent anions derived from a water-soluble anionic polymer containing at least a monovalent salt of alginic acid and having monovalent anionic substituents by using as crosslinking agents two or more salt compounds containing at least divalent metal cations within a medium in which particles of the water-soluble anionic polymer have been dispersed to a concentration of at least 5 wt%.

The water-soluble anionic polymer particles can be produced by, for example, spray-drying a solution that includes a water-soluble anionic polymer containing at least a monovalent salt of alginic acid and thereby inducing particle formation.

The solvent used in this solution is preferably water, a hydrophilic organic solvent or a mixed solvent of water and a hydrophilic organic solvent. Examples of the water include tap water, deionized water and distilled water. Examples of the hydrophilic organic solvent include methanol, ethanol, 1-propanol, 2-propanol, ethylene glycol, propylene glycol, butylene glycol, dipropylene glycol, methyl cellosolve, ethyl cellosolve, propyl cellosolve, diethylene glycol monobutyl ether, methyl cellosolve acetate, ethyl cellosolve acetate, methyl carbitol, ethyl carbitol, butyl carbitol, ethyl carbitol acetate, formic acid, acetic acid, propionic acid, acetone, tetrahydrofuran, dimethylformamide, dimethylsulfoxide, trioxane, tetrahydrofuran, N-methyl-2-pyrrolidone, dimethylamine, monoethanolamine, pyridine and acetonitrile. These may be used singly, or two or more may be used in admixture.

If necessary, a hydrophobic organic solvent may also be added to the above solvent. Examples of the hydrophobic organic solvent include higher alcohols such as 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 1-hexanol, 2-methyl-1-pentanol, 4-methyl-2-pentanol, 2-ethylbutanol, 1-heptanol, 2-heptanol, 3-heptanol, 2-octanol, 2-ethyl-1-hexanol, benzyl alcohol and cyclohexanol; ether alcohols such as butyl cellosolve; ketones such as methyl ethyl ketone, methyl isobutyl ketone and cyclohexanone; esters such as ethyl acetate, butyl acetate, ethyl propionate and cellosolve acetate; aliphatic or aromatic hydrocarbons such as pentane, 2-methylbutane, n-hexane, cyclohexane, 2-methylpentane, 2,2-dimethylbutane, 2,3-dimethylbutane, heptane, n-octane, isooctane, 2,2,3-trimethylpentane, decane, nonane, cyclopentane, methylcyclopentane, methylcyclohexane, ethylcyclohexane, p-menthane, dicyclohexyl, benzene, toluene, xylene and ethylbenzene; halogenated hydrocarbons such as carbon tetrachloride, trichloroethylene, chlorobenzene and tetrabromoethane; ethers such as diethyl ether and dimethyl ether; acetals such as methylal and diethyl acetal; the following cyclic, linear or branched silicone oils and copolymers thereof: dimethylpolysiloxanes such as hexamethylcyclotrisiloxane (D3), octamethylcyclotetrasiloxane (D4), decamethylcyclopentasiloxane (D5), dodecamethylcyclohexasiloxane (D6), tetradecamethylcycloheptasiloxane (D7), hexamethyldisiloxane, octamethyltrisiloxane and decamethyltetrasiloxane, and also methyl trimethicone, methyl phenyl polysiloxane, diphenyl polysiloxane, diphenyl siloxyphenyl trimethicone, trimethyl siloxyphenyl dimethicone, caprylyl methicone and cetyl dimethicone; and sulfur or nitrogen-containing organic compounds such as nitropropene and nitrobenzene.

In this invention, "hydrophilic organic solvent" refers to an organic solvent for which an equal volume mixture with water maintains a uniform appearance. Also, "hydrophobic organic solvent" refers to a solvent for which, after being gently mixed with the same volume of pure water at one atmosphere ($1.013 \times 10^5$ Pa) and a temperature of 20°C and allowed to quiet down, the mixture is unable to maintain a uniform appearance.

The water-soluble anionic polymer particles obtained by the above method are dispersed in water, a hydrophilic organic solvent, or a mixed solvent of water and a hydrophilic organic solvent to a concentration of 5 wt% or more. Following dispersion, crosslinking treatment is carried out using two or more salt compounds containing at least divalent metal cations.

Method 2 is a method that includes the step of crosslinking water-soluble polymeric polyvalent anions derived from a water-soluble anionic polymer containing at least a monovalent salt of alginic acid and having monovalent anionic substituents by using as crosslinking agents two or more salt compounds containing at least divalent metal cations within (B) a medium in which, using water and an oily medium, the water-soluble anionic polymer has been suspended or emulsified in water to a concentration of at least 5 wt%.

Specific examples of the oily medium include the same as those mentioned as examples of above-described hydrophobic organic solvents.

The method for suspending or emulsifying the water-soluble anionic polymer in water is not particularly limited. For example, the method may involve charging a vessel all at once with the water-soluble anionic polymer, water, an oily medium and, if necessary, a surfactant and other ingredients, and using an agitator or a homogenizer to effect suspension or emulsification. Alternatively, the method may involve mixing a solution obtained by dissolving the water-soluble anionic polymer in water or a mixed solvent of water and a hydrophilic organic solvent together with a hydrophobic organic solvent, and using an agitator or a homogenizer to effect suspension or emulsification.

In Method 2, crosslinking treatment is carried out using two or more salt compounds having at least divalent metal cations by adding the two or more salt compounds a plurality of times in divided portions to the above suspension or emulsion (water-in-oil (W/O) emulsion).

Method 3 is a method that includes the step of crosslinking water-soluble polymeric polyvalent anions derived from a water-soluble anionic polymer containing at least a monovalent salt of alginic acid and having monovalent anionic substituents by using as crosslinking agents two or more salt compounds containing at least divalent metal cations within a medium in which the water-soluble anionic polymer has been hydrophilized or dissolved to a concentration of at least 5 wt%.

The solvent that hydrophilizes or dissolves the water-soluble anionic polymer is preferably water, a hydrophilic organic solvent, or a mixed solvent of water and a hydrophilic organic solvent. The water and the hydrophilic organic solvent are exemplified in the same way as in the above explanation of Method 1.

In each of Methods 1 to 3, the crosslinking treatment preferably carries out a crosslinking reaction by independently adding at least one of the two or more salt compounds; specifically, by adding the metal cation-containing salt compounds in order, starting with that for which the amount of addition is smallest. Alternatively, it is preferable to collectively add all the salt compounds other than that for which the amount of addition is largest, and to finish by independently adding the metal cation-containing salt compound for which the amount of addition is largest. By doing so, marine biodegradable polymer particles having metal cation proportions close to the design metal cation proportions can be obtained, enabling the target marine biodegradable polymer particles to be efficiently and stably obtained.

If necessary, crosslinking treatment may be carried out while heating. The heating temperature is preferably between 10°C and 100°C, and more preferably between 15°C and 80°C. The treatment time is preferably from 0.5 to 24 hours, and more preferably from 1 to 12 hours. With heating, the viscosity of the solvent decreases, facilitating impregnation of metal cations to the interior of the particles.

The resulting marine biodegradable polymer particles may be optionally subjected to surface treatment or grinding treatment with known equipment.

In cases where the marine biodegradable polymer particles are subjected to hydrophobizing treatment using a hydrophobizing agent, the method for doing so may involve carrying out hydrophobizing treatment on the marine biodegradable polymer particles obtained by the above-described method, or may involve carrying out hydrophobizing treatment at the same time as crosslinking in above Methods 1 to 3. The hydrophobizing agents mentioned above may be used here as the hydrophobizing agent.

An exemplary method for hydrophobizing marine biodegradable polymer particles involves dissolving a hydrophobizing agent in a solvent, and adding and dispersing the marine biodegradable polymer particles within the resulting solution, thereby causing the hydrophobizing agent to deposit on the particle surfaces or on both the surfaces and interior of the particles.

The method used to carry out hydrophobizing treatment at the same time as crosslinking in Method 1 may involve spray-drying a solution which includes both a water-soluble anionic polymer containing at least a monovalent salt of alginic acid and the hydrophobizing agent so as to form particles, and carrying out crosslinking treatment by the above-described method. In this way, hydrophobizing treatment takes place at the same time.

The method used to carry out hydrophobizing treatment at the same time as crosslinking in Method 2 may involve adding the hydrophobizing agent to the above-described suspension or emulsion, and subjecting the resulting mixture to crosslinking treatment by the above-described method. In this way, hydrophobizing treatment takes place at the same time.

The method used to carry out hydrophobizing treatment at the same time as crosslinking in Method 3 may involve adding the hydrophobizing agent to the medium, and subjecting the resulting mixture to crosslinking treatment by the above-described method. In this way, hydrophobizing treatment takes place at the same time.

For specific hydrophilizing treatment methods, reference can be made to the methods described in paragraphs [0055] to [0095] of JP-A 2020-125256, the methods described in paragraphs [0056] to [0100] of JP-A 2021-195321, and the methods described in paragraphs [0061] to [0112] of JP-A 2021-191810.

[Resin Composition]

Resin compositions whose biodegradation in the oceans is accelerated can be obtained by using the marine biodegradable polymer particles of the invention in combination with a resin, particularly a biodegradable resin. A plurality of resins may be used in combination for the purpose of adjusting the physical properties and handleability of the resin composition. As used herein, "biodegradable resin" refers to a resin which decomposes under the action of microorganisms in the natural world, ultimately breaking down to inorganic substances such as water and carbon dioxide.

Examples of resins that may be combined with the marine biodegradable polymer particles of the invention include polyethylene, polyester, polypropylene, polyethylene terephthalate, vinyl chloride, polystyrene, polyurethane, epoxy resins, chlorinated polyethylene resins, chlorinated polypropylene resins, modified nylon resins, phenolic resins, silicone resins, polyvinyl acetate, ethylene-vinyl acetate copolymers, polyvinyl chloride, polyvinylidene chloride, styrene-maleic acid resins, styrene-butadiene resins, butadiene resins, acrylonitrile-butadiene resins, poly(meth)acrylonitrile resins, (meth)acrylamide resins, bioPET, biopolyamides, biopolycarbonates, biopolyurethanes, polyvinyl alcohols, polybutylene adipate/terephthalate, polyethylene terephthalate succinate, biopolybutylene succinate, polylactic acid blends, starch-blended polyester resins, polybutylene terephthalate succinate, polylactic acid and polyhydroxyalkanoates. From the standpoint of reducing the impact on the environment, resins having a high biodegradability are especially preferred.

Examples of such biodegradable resins include resins obtained from raw materials derived from petroleum, such as polycaprolactone, poly(caprolactone/butylene succinate), polybutylene succinate, polyethylene terephthalate copolymers, poly(ethylene terephthalate/succinate), poly(butylene adipate/terephthalate), poly(tetramethylene adipate/terephthalate), polyethylene succinate, poly(butylene succinate/carbonate), polyvinyl alcohol, polyglycolic acid, glycolic acid/caprolactone copolymers and glycolic acid/trimethylene carbonate copolymers; resins obtained from raw materials which are partially derived from biomass, such as (polylactic acid/polybutylene succinate-based) block copolymers, (polylactic acid/polycaprolactone) copolymers, (polylactic acid/polyether) copolymers, polylactic acid-blended PBAT, lactic acid/glycolic acid copolymers, biopolybutylene succinate, poly(butylene succinate/adipate), starch-blended polyester resins and poly(butylene terephthalate succinate); resins obtained from raw materials which are 100% derived from biomass, including polyhydroxyalkanoates such as polyhydroxybutyrate, polyhydroxyvalerate, polyhydroxycaprylate and poly(hydroxybutyrate/hydroxyhexanoate), and also polylactic acid (PLA); and resins derived from naturally occurring macromolecules such as cellulose, cellulose acetate, cellulose ester resins, starches, esterified starches and chitosan. From the standpoint of lowering the environmental impact, it is preferable for the raw materials for the resin that is combined with the marine biodegradable polymer particles of the invention to be derived from biomass, and most preferable for the raw materials to be 100% derived from biomass.

The resin composition of the invention may include a solvent. The solvent may be one which dissolves the resin serving as the matrix while leaving the marine biodegradable polymer particles undissolved as particles, or may be one which dissolves both the resin and the marine biodegradable polymer particles. By suitably adjusting these ingredients, the resin composition may be used as a formed body obtained by film formation via casting or the like, or may be used as, for example, a coating, ink or surface treatment agent. Examples of preferred solvents include water, hexane, heptane, N-methylpyrrolidone, dimethylformamide, dimethylacetamide, dimethylsulfoxide, dimethylsulfone, acetone, methyl ethyl ketone, diethyl ketone, acetophenone, dimethyl ether, dipropyl ether, tetrahydrofuran, chloroform, methylene chloride, trichloroethylene, ethylene dichloride, dichloroethane, tetrachloroethane, chlorobenzene, methanol, ethanol, n-propanol, isopropanol, butanol, pentanol, methyl glycol, methyl triglycol, hexyl glycol, phenyl glycol, ethylene glycol, propylene glycol, phenol, cresol, polyethylene glycol, benzene, toluene and xylene. One of these may be used alone or two or more may be used in admixture.

When using a solvent, the total concentration of resin and marine biodegradable polymer particles in the resin composition is preferably from 0.5 to 90 wt%, more preferably from 1 to 80 wt%, even more preferably from 5 to 60 wt%, and most preferably from 10 to 50 wt%. The proportion of marine biodegradable polymer particles relative to the resin, expressed as a weight ratio, is preferably from 99:1 to 10:90, more preferably from 97:3 to 40:60, even more preferably from 95:5 to 50:50, and most preferably from 90: 10 to 60:40.

The resin composition of the invention does not have to include a solvent. In this case, the resin may be heat-melted and marine biodegradable polymer particles that do not melt therein may be added to and mixed with the molten resin, or the resin and the marine biodegradable polymer particles may both be heat-melted and mixed together. In cases where the marine biodegradable polymer particles are heat-melted and mixed together with the resin, it is preferable for the marine biodegradable polymer particles to have a softening point or melting point suitable for the melting temperature of the resin. The lower limit of this softening point or melting point, in order of increasing preference, is at least 60°C, at least 80°C, at least 100°C, or at least 120°C. The upper limit, in order of increasing preference, is 300°C or less, 250°C or less, 200°C or less, or 180°C or less.

To obtain a sufficient degradation promoting effect and not adversely affect the physical properties of the resin composition, it is desirable for the resin composition of the invention to have a content of marine biodegradable polymer particles per 100 parts by weight of the resin with a lower limit which, in order of increasing preference, is at least 1 part by weight, at least 2 parts by weight, at least 3 parts by weight, or at least 5 parts by weight. The upper limit in the content of biodegradability accelerator, in order of increasing preference, is 100 parts by weight or less, 80 parts by weight or less, 50 parts by weight or less, or 30 parts by weight or less. Particularly in cases where the marine biodegradable polymer particles are present in a particulate form, the content lower limit is preferably at least 1 part by weight, and more preferably at least 3 parts by weight, and the content upper limit is preferably 50 parts by weight or less, and more preferably 30 parts by weight or less.

The resin composition of the invention may optionally include additives such as antioxidants, parting agents, release agents, surface modifiers, hydrophobizing agents, water repelling agents, hydrophilizing agents, dyes and pigments, colorants, heat stabilizers, light stabilizers, weatherability enhancers, antistatic agents, anti-fogging agents, lubricants, anti-blocking agents, hardeners, softeners, compatibilizers, flame retardants, flow enhancers, plasticizers, dispersants, antimicrobial agents, fillers and metal inactivators. The content of these additives, although not particularly limited so long as there is no loss in the advantageous effects of the invention, is preferably from about 0.1 to about 50 parts by weight per 100 parts by weight of the resin.

In cases where the resin composition includes a solvent, the composition can be prepared by, for example, adding the resin, the marine biodegradable polymer particles and the optional additives to the solvent, either at the same time or in any order, and mixing together the ingredients. Alternatively, in cases where the resin composition does not include a solvent, the resin may be melted and the marine biodegradable polymer particles and the optional additives may be added thereto, either at the same time or in any order, and mixed together; or the resin and the marine biodegradable polymer particles may be heated, melted together and mixed, and the optional additives then added and mixed therewith.

[Formed Body]

A formed body composed of marine biodegradable polymer particles dispersed or dissolved in the resin can be obtained by molding or forming the resin composition. When the resin composition includes a solvent, molding or forming may be carried out using the resin composition directly as is. When the resin composition does not include a solvent, molding or forming may be carried out after heating and melting the resin, or both the resin and the marine biodegradable polymer particles, within the resin composition.

The formed body may be in the shape of a film, fibers, a sheet or an expansion-molded body, or may have some other shape according to the intended use. The molding or forming method is not particularly limited; use can be made of various methods known to the art. Examples of such methods include blow molding, injection molding, extrusion, compression molding, melt extrusion, film casting and calendering.

[Surface Modifier]

Because they dissolve in salt water, the marine biodegradable polymer particles of the invention can be used as a hole-forming agent for producing a porous body or as a surface modifier. For example, when a formed body obtained from a resin composition containing the marine biodegradable polymer particles is immersed in an aqueous solution of a monovalent metal salt such as sodium chloride or potassium chloride, surface modification of only the surface layer is possible; when impregnation is continued further, the marine biodegradable polymer particles dissolve, enabling a porous body to be produced. By applying these characteristics, use can be expanded to, for example, surfactants, coatings and inks.

[Uses of Marine Biodegradable Polymer Particles]

The marine biodegradable polymer particles of the invention can be dispersed in water, a hydrophilic organic solvent, a hydrophobic organic solvent or a mixed solvent of these, and used as a dispersion.

The marine biodegradable polymer particles of the invention can be used as an additive in liquids, paint films, plastic film, plate materials and molded paper products. For example, they can be widely used in light-scattering agents and optical filter materials, colorants, cosmetics, absorbents, adsorbents, inks, adhesives, electromagnetic shielding materials, fluorescence sensors, biomarkers, recording materials, recording elements, polarizing materials, drug carriers for drug delivery systems (DDS), biosensors, DNA chips, diagnostic agents, thermally porosified moldings and anti-blocking agents.

The blocking of light or ultraviolet rays (UV) from entering a room, vehicle or the like by means of, for example, window glass products or interior goods such as curtains and wall materials serves not only to protect the human body from sunburns and other adverse effects but is useful as well by making it possible to prevent the deterioration of decorative articles, etc. within the room or vehicle.

The marine biodegradable polymer particles of the invention are suitable as an additive for personal care products and cosmetics. In such cases, it is preferable for the marine biodegradable polymer particles of the invention to be added as, for example, a scrubbing agent, filler, thickener, texture modifier or film-forming agent.

The inventive marine biodegradable polymer particles, being derived chiefly from a naturally occurring polymer, can be employed in liquid systems and other applications having a high liquid ingredient content while enhancing, for example, the lightweight properties, tactile feel, flow properties and solution dispersibility. Also, when the particle shape has recesses, the particles possess, on account of their distinctive shape, adhesive forces which differ from those of ordinary spherical particles. As a result, along with enabling improved light scattering properties, etc. to be obtained, the inventive particles are effective for improving the bonding strength of pressed compacts of foundation or the like and also the holding power following application. In addition, the optical properties of the particles make the skin appear lighter and can enhance the covering power due to a shading effect. Also, on account of the distinctive slip characteristics and optical characteristics attributable to the shape of the particles and the fact that they include a plurality of metal cations, spread over the skin is excellent, wrinkles and pores can be made inconspicuous by finely filling in furrows in the skin texture, and the flowability and light-diffusing ability of the overall product are freely controllable. The amount of addition, based on the overall product composition, is preferably from 0.1 to 50 wt%, and more preferably from 0.5 to 30 wt%. Light scattering properties such as the UV scattering effect and the shading effect, and flowability, moldability, improvements in adhesion, the finished look and other qualities can be suitably adjusted according to the intended use or purpose. Based on investigations by the inventors, as an additive for cosmetics, the inclusion of from 1 to 20 wt% is especially preferred. These particles may also be suitably adjusted and used in combination with commercially available particles.

Cosmetics in which the inventive particles provide highly advantageous effects include, in particular, skin care products, hair products, antiperspirants, makeup products, UV protection products and scented products. Specific examples include base cosmetics such as milky emulsions, creams, lotions, calamine lotions, sunscreens, makeup bases, suntan lotions, aftershave lotions, pre-shave lotions, packs, cleansing materials, facial cleansers, cosmetics for acne, and essences; makeup cosmetics such as foundation, face powder, mascara, eye shadow, eyeliner, eyebrow, cheek, nail color, lip cream and lipstick; and also shampoos, rinses, conditioners, hair colors, hair tonics, setting agents, body powders, hair growth promoters, deodorants, depilatories, soaps, body shampoos, bath preparations, hand soaps and perfumes. The form of the product is not particularly limited and may be, for example, a liquid, emulsion, cream, solid, paste, gel, powder, multi-layer preparation, mousse or spray. The marine biodegradable polymer particles of the invention can be expected to have useful effects as an additive for these cosmetics.

The marine biodegradable polymer particles of the invention can be included as an additive in printing inks used in, for example, screen printing, offset printing, process printing, gravure printing, pad printing, coaters and inkjet printing; as an additive in inks for writing implements such as felt-tip markers, ballpoint pens, fountain pens, calligraphy pens and magic markers; and as an additive for stationery supplies such as crayons, paints and erasers.

The marine biodegradable polymer particles of the invention are suitable as an additive for paints and coatings employed in, for example, brush painting, spray painting, electrostatic coating, electrodeposition coating, flow coating, roller coating and dip coating. For example, they are suitable as an additive for paints and coatings that may be used on transportation equipment such as automobiles, railway cars, helicopters, ships, bicycles, snowmobiles, ropeways, lifts, hovercrafts and motorcycles; building members such as window sashes, shutters, cisterns, doors, balconies, outside panels for construction, roofing, staircases, skylights and concrete walls; the exterior walls and interior finish on the inside and outside of buildings; roadway members such as guardrails, pedestrian bridges, sound insulating walls, road signs, highway sidewalls, elevated railroad bridges, and bridges; industrial plant members such as tanks, pipes, towers and smokestacks; agricultural facilities such as PVC and other types of greenhouses, silos and agricultural sheeting; telecommunications facilities such as utility poles, transmission towers and parabolic antennas; electrical equipment such as electrical service boxes, lighting equipment, outdoor air conditioning units, washing machines, refrigerators and microwave ovens, as well as covers for these; and other articles such as monuments, gravestones, paving materials, windscreens, waterproof sheeting and curing sheets for construction.

The form of the paint or coating is exemplified by solvent-based paints and also aqueous dispersion paints, nonaqueous dispersion paints, powder paints and electrodeposition coatings, and may be suitably selected as needed.

EXAMPLES

Production Examples, Examples of the invention and Comparative Examples are given below by way of illustration, although the invention is not limited to these Examples. In the following Examples of the invention and Comparative Examples, the amounts and ratios in which the metal cations are included were measured by ICP-MS (ICPE-9820, from Shimadzu Corporation). The particle size distributions and volume mean particle sizes (MV) were measured using the MICROTRACK MT3000 (Nikkiso Co., Ltd.). The sodium alginate and potassium alginate used in the Production Examples and Examples of the invention were as follows.

- Sodium alginate: available under the trade name "Kimika Algin ULV-L3" from Kimika Corporation (viscosity as a 10 wt% aqueous solution, 40 mPa·s)

- Potassium alginate: available under the trade name "Kimika Algin K-ULV-L3" from Kimika Corporation (viscosity as a 10 wt% aqueous solution, 40 mPa·s)

[1] Production of Marine Biodegradable Polymer Particles

[Production Example 1] Production of Uncrosslinked Polymer Particles A1

The ingredients shown below were charged into a 5,000 mL heatable vessel and dispersed using an agitator.

| | |
|---|---|
| Sodium alginate | 400.0 g |
| Deionized water | 4,000.0 g |

The contents were then heated to 60°C and the sodium alginate was dissolved over 2 hours, preparing a 9.0 wt% aqueous solution. Next, with the solids maintained in a dissolved state, the resulting aqueous solution was spray-dried (hot-air temperature, 200°C) using a spray dryer, giving Uncrosslinked Polymer Particles A1. The resulting particles were examined by scanning electron microscopy (SEM) and the particle shapes checked, whereupon the particles were found to be approximately spherical particles with recesses. The particles were monodispersed particles having, in the particle size distribution, a volume mean diameter (MV) of 6 $\mu$m.

[Production Example 2] Production of Uncrosslinked Polymer Particles A2

The ingredients shown below were charged into a 5,000 mL heatable vessel and dispersed using an agitator.

| | |
|---|---|
| Sodium alginate | 256.0 g |
| Sodium CMC | 64.0 g |
| Deionized water | 4,260.0 g |

The contents were then heated to 60°C and the sodium alginate and sodium CMC were dissolved over 2 hours, preparing a 7.0 wt% aqueous solution. Next, with the solids maintained in a dissolved state, the resulting aqueous solution was spray-dried (hot-air temperature, 200°C) using a spray dryer, giving Uncrosslinked Polymer Particles A2. The resulting particles A2 were examined by SEM and the particle shapes checked, whereupon the particles were found to be approximately spherical particles with recesses. The particles were monodispersed particles having, in the particle size distribution, a MV of 5 $\mu$m.

[Production Example 3] Production of Uncrosslinked Polymer Particles A3

The ingredients shown below were charged into a 2,000 mL heatable vessel and dispersed using an agitator.

| | |
|---|---|
| Sodium alginate | 57.0 g |
| Sodium hyaluronate | 3.0 g |
| Deionized water | 798.0 g |

The contents were then heated to 60°C and the sodium alginate and sodium hyaluronate were dissolved over 2 hours, preparing a 7.0 wt% aqueous solution. Next, with the solids maintained in a dissolved state, the resulting aqueous solution was spray-dried (hot-air temperature, 200°C) using a spray dryer, giving Uncrosslinked Polymer Particles A3. The resulting particles were examined by SEM and the particle shapes checked, whereupon the particles were found to be spherical. The particles were monodispersed particles having, in the particle size distribution, a MV of 30 $\mu$m.

[Production Example 4] Production of Uncrosslinked Polymer Particles A4

The ingredients shown below were charged into a 10,000 mL heatable vessel and dispersed using an agitator.

| | |
|---|---|
| Potassium alginate | 304.0 g |
| Sodium palmitate | 16.0 g |
| Deionized water | 4,250.0 g |

The contents were then heated to 60°C and the potassium alginate and sodium palmitate were dissolved over 2 hours, preparing a 7.0 wt% aqueous solution. Next, with the solids maintained in a dissolved state, the resulting aqueous solution was spray-dried (hot-air temperature, 200°C) using a spray dryer, giving Uncrosslinked Polymer Particles A4. The resulting particles were examined by SEM and the particle shapes checked, whereupon the particles were found to be approximately spherical particles with recesses. The particles were monodispersed particles having, in the particle size distribution, a MV of 15 $\mu$m.

[Example 1-1] Production of Marine Biodegradable Polymer Particles AC-1

The ingredients shown below were charged into a 300 mL flask, and a 30 wt% alcohol dispersion of Uncrosslinked Polymer Particles A1 was prepared using a stirrer.

| | |
|---|---|
| Uncrosslinked Polymer Particles A1 | 30.0 g |
| Isopropyl alcohol (IPA) | 70.0 g |

Next, 9.4 g of a 40 wt% aqueous solution of magnesium chloride was added dropwise to the alcohol dispersion during stirring. Following the completion of addition, stirring was continued for one hour, thereby carrying out primary crosslinking treatment. Following primary crosslinking treatment, 18.8 g of a 40 wt% aqueous solution of calcium chloride was slowly poured into the alcohol dispersion and stirring was carried out for 15 minutes. Next, 50.0 g of purified water was added dropwise and the mixture was stirred for 2 hours, thereby carrying out secondary crosslinking treatment. Following the completion of stirring, filtration and washing were repeatedly carried out using deionized water and the final dispersion was rendered into a powder with a freeze dryer, giving the target Marine Biodegradable Polymer Particles AC-1.
The Marine Biodegradable Polymer Particles AC-1 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed. According to ICP-MS analysis, the content of magnesium and calcium was 8.2 wt% and the content ratio therebetween was Mg : Ca = 1: 1.9.

[Example 1-2] Production of Marine Biodegradable Polymer Particles AC-2

Aside from changing the 9.4 g of a 40 wt% aqueous solution of magnesium chloride to 9.4 g of a 40 wt% mixed aqueous solution of magnesium chloride and strontium chloride (weight ratio of magnesium chloride and strontium chloride, 2:3), the target Marine Biodegradable Polymer Particles AC-2 were obtained in the same way as in Example 1-1.
The Marine Biodegradable Polymer Particles AC-2 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed. According to ICP-MS analysis, the content of magnesium, strontium and calcium was 11.4 wt% and the content ratio therebetween was Mg : Sr : Ca = 1:3:5.7.

[Example 1-3] Production of Marine Biodegradable Polymer Particles AC-3

Aside from changing the 9.4 g of a 40 wt% aqueous solution of magnesium chloride to 9.4 g of a 40 wt% aqueous solution of zinc chloride, the target Marine Biodegradable Polymer Particles AC-3 were obtained in the same way as in Example 1-1.

The Marine Biodegradable Polymer Particles AC-3 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed. According to ICP-MS analysis, the content of zinc and calcium was 12.2 wt% and the content ratio therebetween was Zn : Ca = 1:1.3.

[Example 1-4] Production of Marine Biodegradable Polymer Particles AC-4

The ingredients shown below were charged into a 300 mL flask, and a 30 wt% alcohol dispersion of Uncrosslinked Polymer Particles A1 was prepared using a stirrer.

| | |
|---|---|
| Uncrosslinked Polymer Particles A1 | 30.0 g |
| IPA | 70.0 g |

Next, 7.5 g of a 40 wt% aqueous solution of magnesium chloride was added dropwise to the alcohol dispersion during stirring. Following the completion of addition, stirring was continued for one hour, thereby carrying out primary crosslinking treatment. Following primary crosslinking treatment, 22.5 g of a 40 wt% aqueous solution of strontium chloride was slowly poured into the alcohol dispersion and stirring was carried out for 15 minutes. Next, 50.0 g of purified water was added dropwise and the mixture was stirred for 2 hours, thereby carrying out secondary crosslinking treatment. Following the completion of stirring, filtration and washing were repeatedly carried out using deionized water and the final dispersion was rendered into a powder with a freeze dryer, giving the target Marine Biodegradable Polymer Particles AC-4.

The Marine Biodegradable Polymer Particles AC-4 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed. According to ICP-MS analysis, the content of magnesium and strontium was 15.8 wt% and the content ratio therebetween was Mg : Sr = 1:5.5.

[Example 1-5] Production of Marine Biodegradable Polymer Particles AC-5

Aside from changing the 7.5 g of a 40 wt% aqueous solution of magnesium chloride to 7.5 g of a 40 wt% aqueous solution of zinc chloride, the target Marine Biodegradable Polymer Particles AC-5 were obtained in the same way as in Example 1-4.

The Marine Biodegradable Polymer Particles AC-5 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed. According to ICP-MS analysis, the content of zinc and strontium was 20.5 wt% and the content ratio therebetween was Zn : Sr = 1:3.5.

[Example 1-6] Production of Marine Biodegradable Polymer Particles AC-6

The ingredients shown below were charged into a 300 mL flask, and a 30 wt% alcohol dispersion of Uncrosslinked Polymer Particles A1 was prepared using a stirrer.

| | |
|---|---|
| Uncrosslinked Polymer Particles A1 | 30.0 g |
| IPA | 70.0 g |

Next, 5.65 g of a 40 wt% aqueous solution of magnesium chloride was added dropwise to the alcohol dispersion during stirring. Following the completion of addition, stirring was continued for one hour, thereby carrying out primary crosslinking treatment. After primary crosslinking treatment, 5.65 g of a 40 wt% aqueous solution of strontium chloride was added dropwise to the alcohol dispersion. Following the completion of addition, stirring was continued for 1 hour, thereby completing secondary crosslinking treatment. After secondary crosslinking treatment, 16.9 g of a 40 wt% aqueous solution of calcium chloride was slowly poured into the alcohol dispersion and stirring was carried out for 15 minutes. Next, 50.0 g of purified water was added dropwise and the mixture was stirred for 2 hours, thereby carrying out tertiary crosslinking treatment. Following the completion of stirring, filtration and washing were repeatedly carried out using deionized water and the final dispersion was rendered into a powder with a freeze dryer, giving the target Marine Biodegradable Polymer Particles AC-6.

The Marine Biodegradable Polymer Particles AC-6 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed. According to ICP-MS analysis, the content of magnesium, calcium and strontium was 11.0 wt% and the content ratio therebetween was Mg : Ca : Sr = 1:3.2:2.2.

[Example 1-7] Production of Marine Biodegradable Polymer Particles AC-7

The ingredients shown below were charged into a 300 mL flask, and a 30 wt% alcohol dispersion of Uncrosslinked Polymer Particles A1 was prepared using a stirrer.

| | |
|---|---|
| Uncrosslinked Polymer Particles A1 | 30.0 g |
| IPA | 70.0 g |

Next, 5.25 g of a 40 wt% aqueous solution of magnesium chloride was added dropwise to the alcohol dispersion during stirring. Following the completion of addition, stirring was continued for one hour, thereby carrying out primary crosslinking treatment. After primary crosslinking treatment, 3.00 g of a 30 wt% aqueous solution of aluminum chloride was added dropwise to the alcohol dispersion. Following the completion of addition, stirring was continued for 1 hour, thereby completing secondary crosslinking treatment. After secondary crosslinking treatment, 13.15 g of a 40 wt% aqueous solution of calcium chloride was slowly poured into the alcohol dispersion and stirring was carried out for 15 minutes. Next, 50.0 g of purified water was added dropwise and the mixture was stirred for 2 hours, thereby carrying out tertiary crosslinking treatment. Following the completion of stirring, filtration and washing were repeatedly carried out using deionized water and the final dispersion was rendered into a powder with a freeze dryer, giving the target Marine Biodegradable Polymer Particles AC-7.

The Marine Biodegradable Polymer Particles AC-7 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed. According to ICP-MS analysis, the content of magnesium, aluminum and calcium was 8.4 wt% and the content ratio therebetween was Mg : Al : Ca = 2.9:1:11.4.

[Example 1-8] Production of Marine Biodegradable Polymer Particles AC-8

The ingredients shown below were charged into a 300 mL flask, and a 30 wt% alcohol dispersion of Uncrosslinked Polymer Particles A2 was prepared using a stirrer.

| | |
|---|---|
| Uncrosslinked Polymer Particles A2 | 30.0 g |
| IPA | 70.0 g |

Next, 9.4 g of a 40 wt% aqueous solution of magnesium chloride was added dropwise to the alcohol dispersion during stirring. Following the completion of addition, stirring was continued for one hour, thereby carrying out primary crosslinking treatment. After primary crosslinking treatment, 18.8 g of a 40 wt% aqueous solution of calcium chloride was slowly poured into the alcohol dispersion and stirring was carried out for 15 minutes. Next, 50.0 g of purified water was added dropwise and the mixture was stirred for 2 hours, thereby carrying out secondary crosslinking treatment. Following the completion of stirring, filtration and washing were repeatedly carried out using deionized water and the final dispersion was rendered into a powder with a freeze dryer, giving the target Marine Biodegradable Polymer Particles AC-8.

The Marine Biodegradable Polymer Particles AC-8 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed. According to ICP-MS analysis, the content of magnesium and calcium was 8.0 wt% and the content ratio therebetween was Mg : Ca = 1: 1.8.

[Example 1-9] Production of Marine Biodegradable Polymer Particles AC-9

The ingredients shown below were charged into a 300 mL flask, and a 30 wt% alcohol dispersion of Uncrosslinked Polymer Particles A3 was prepared using a stirrer.

| | |
|---|---|
| Uncrosslinked Polymer Particles A3 | 30.0 g |
| IPA | 70.0 g |

Next, 7.5 g of a 40 wt% aqueous solution of magnesium chloride was added dropwise to the alcohol dispersion during stirring. Following the completion of addition, stirring was continued for one hour, thereby carrying out primary crosslinking treatment. After primary crosslinking treatment, 20.65 g of a 40 wt% aqueous solution of strontium chloride was slowly poured into the alcohol dispersion and stirring was carried out for 15 minutes. Next, 50.0 g of purified water was added dropwise and the mixture was stirred for 2 hours, thereby carrying out secondary crosslinking treatment. Following the completion of stirring, filtration and washing were repeatedly carried out using deionized water and the final dispersion was rendered into a powder with a freeze dryer, giving the target Marine Biodegradable Polymer Particles AC-9.
The Marine Biodegradable Polymer Particles AC-9 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed. According to ICP-MS analysis, the content of magnesium and strontium was 15.5 wt% and the content ratio therebetween was Mg : Sr = 1:5.8.

[Example 1-10] Production of Marine Biodegradable Polymer Particles AC-10

The ingredients shown below were charged into a 300 mL flask, and a 30 wt% alcohol dispersion of Uncrosslinked Polymer Particles A4 was prepared using a stirrer.

| | |
|---|---|
| Uncrosslinked Polymer Particles A4 | 30.0 g |
| IPA | 70.0 g |

Next, 7.5 g of a 40 wt% aqueous solution of zinc chloride was added dropwise to the alcohol dispersion during stirring. Following the completion of addition, stirring was continued for one hour, thereby carrying out primary crosslinking treatment. After primary crosslinking treatment, 20.65 g of a 40 wt% aqueous solution of strontium chloride was slowly poured into the alcohol dispersion and stirring was carried out for 15 minutes. Next, 50.0 g of purified water was added dropwise and the mixture was stirred for 2 hours, thereby carrying out secondary crosslinking treatment. Following the completion of stirring, filtration and washing were repeatedly carried out using deionized water and the final dispersion was rendered into a powder with a freeze dryer, giving the target Marine Biodegradable Polymer Particles AC-10.
The Marine Biodegradable Polymer Particles AC-10 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed. According to ICP-MS analysis, the content of zinc and strontium was 18.7 wt% and the content ratio therebetween was Zn : Sr = 1:3.3.

[Example 1-11] Production of Marine Biodegradable Polymer Particles AC-11

The ingredients shown below were charged into a 5,000 mL heatable vessel and emulsified by 5 minutes of agitation using a homogenizer (IKA, model T 25).

| Sodium alginate | 200.0 g |
|---|---|
| Deionized water | 800.0 g |
| Hexane | 1,000.0 g |
| Sorbitan monooleate | 5.0 g |

Next, 62.5 g of a 40 wt% aqueous solution of magnesium chloride was added dropwise to the emulsion. Following the completion of addition, agitation was continued for one hour, thereby carrying out primary crosslinking treatment. Next, 125.0 g of a 40 wt% aqueous solution of calcium chloride was added dropwise. After the completion of addition, agitation was carried out for 2 hours at 50°C, thereby carrying out secondary crosslinking treatment. Following the completion of cooling, centrifugal washing with ethanol and deionized water was repeatedly carried out and the final aqueous dispersion was rendered into a powder with a freeze dryer, giving the target Marine Biodegradable Polymer Particles AC-11.

The Marine Biodegradable Polymer Particles AC-11 were examined by SEM and the particle shapes checked, whereupon the particles were found to be spherical monodispersed particles having a MV of 10 $\mu$m in the particle size distribution. According to ICP-MS analysis, the content of magnesium and calcium was 8.0 wt% and the content ratio therebetween was Mg : Ca = 1:1.5.

[Example 1-12] Production of Marine Biodegradable Polymer Particles AC-12

The ingredients shown below were charged into a 3,000 mL heatable vessel and were completely dissolved at 60°C using a stirrer.

| Sodium alginate | 190.0 g |
|---|---|
| Sodium N-myristoyl sarcosinate | 10.0 g |
| Deionized water | 1,000.0 g |

Next, 62.5 g of a 40 wt% aqueous solution of magnesium chloride was added dropwise to the solution under high-speed stirring. Following the completion of addition, stirring was continued for one hour, thereby carrying out primary crosslinking treatment. Next, 125.0 g of a 40 wt% aqueous solution of strontium chloride was added dropwise. After the completion of addition, agitation was carried out for 2 hours at 50°C, thereby carrying out secondary crosslinking treatment and causing the particles to completely separate out. Following the completion of stirring, repeated filtration and washing was carried out using deionized water and the particles were dried. The resulting particles were pulverized using a grinding machine (Wonder Blender WB-1, from Osaka Chemical Co., Ltd.), giving the target Marine Biodegradable Polymer Particles AC-12.

The resulting Marine Biodegradable Polymer Particles AC-12 were examined by SEM and the particle shapes checked, whereupon the particles were found to be irregularly shaped particles that were monodispersed and had a MV of 8 $\mu$m in the particle size distribution. According to ICP-MS analysis, the content of magnesium and strontium was 13.4 wt% and the content ratio therebetween was Mg : Sr = 1:3.2.

[Comparative Example 1-1]

The Uncrosslinked Polymer Particles A1 obtained in Production Example 1 were used as Comparative Polymer Particles BC-1.

[Comparative Example 1-2]

The ingredients shown below were charged into a 300 mL flask, and a 30 wt% alcohol dispersion of Uncrosslinked Polymer Particles A1 was prepared using a stirrer.

| Uncrosslinked Polymer Particles A1 | 30.0 g |
|---|---|
| IPA | 70.0 g |

Next, 28.15 g of a 40 wt% aqueous solution of calcium chloride was slowly poured into the alcohol dispersion and the mixture was stirred for 15 minutes, 50.0 g of purified water was added dropwise, and the mixture was subsequently stirred for 2 hours, thereby carrying out crosslinking treatment. Following the completion of stirring, filtration and washing were repeatedly carried out using deionized water and the final dispersion was rendered into a powder with a freeze dryer, giving Comparative Polymer Particles BC-2 that were crosslinked with only calcium.

The Comparative Polymer Particles BC-2 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed.

[Comparative Example 1-3]

The ingredients shown below were charged into a 300 mL flask, and a 30 wt% alcohol dispersion of Uncrosslinked Polymer Particles A1 was prepared using a stirrer.

| Uncrosslinked Polymer Particles A1 | 30.0 g |
| IPA | 70.0 g |

Next, 31.9 g of a 40 wt% aqueous solution of strontium chloride was slowly poured into the alcohol dispersion and the mixture was stirred for 15 minutes, 50.0 g of purified water was added dropwise, and the mixture was subsequently stirred for 2 hours, thereby carrying out crosslinking treatment. Following the completion of stirring, filtration and washing were repeatedly carried out using deionized water and the final dispersion was rendered into a powder with a freeze dryer, giving Comparative Polymer Particles BC-3 that were crosslinked with only strontium.

The Comparative Polymer Particles BC-3 were examined by SEM and the particle shapes checked, whereupon the particles had substantially the same particle diameter as before crosslinking treatment. In addition, the particle size distribution was checked by particle size distribution measurement, whereupon the particles exhibited a distribution similar to that before crosslinking treatment, confirming that the particles had not agglomerated and were monodispersed.

[Comparative Example 1-4]

The ingredients shown below were charged all at once into a 3,000 mL flask, and a suspension was prepared at 800 rpm with a disperser blade. Under a stream of nitrogen gas, the suspension was heated at an oil bath temperature of 80°C and stirred for 8 hours, giving a particle dispersion. Next, the dispersion was subjected to repeated centrifugal separation (5 times) and classification and washing operations were carried out, thereby producing Comparative Polymer Particles BC-4 which were spherical polymer particles of polymethyl methacrylate alone having an average particle diameter of 6 $\mu$m.

| Water | 1,386.5 g |
| Methyl methacrylate | 173.4 g |
| Lauryl peroxide | 8.6 g |
| Polyvinyl pyrrolidone (K-30) | 17.3 g |

The shapes, primary starting materials, at least divalent metals used in crosslinking, contents and content ratios of those metals, maximum differences ($\Delta$R) in atomic radii and MV for Marine Biodegradable Polymer Particles AC-1 to AC-12 and Comparative Polymer Particles BC-1 to BC-4 are shown collectively in Table 1.

[Table 1]

| | Particles | Primary starting material | Crosslinking metal | Metal content (wt%) | Metal content ratio | $\Delta$R (Å) | MV ($\mu$m) |
|---|---|---|---|---|---|---|---|
| Example 1-1 | AC-1 | Na alginate | Mg/Ca | 8.2 | 1/1.9 | 34 | 6 |
| Example 1-2 | AC-2 | Na alginate | Mg/Sr/Ca | 11.4 | 1/3/5.7 | 50 | 6 |
| Example 1-3 | AC-3 | Na alginate | Zn/Ca | 12.2 | 1/1.3 | 54 | 6 |
| Example 1-4 | AC-4 | Na alginate | Mg/Sr | 15.8 | *1/5.5* | 50 | 6 |
| Example 1-5 | AC-5 | Na alginate | Zn/Sr | 20.5 | 1/3.5 | 70 | 6 |
| Example 1-6 | AC-6 | Na alginate | Mg/Ca/Sr | 11.0 | 1/3.2/2.2 | 50 | 6 |

(continued)

| | Particles | Primary starting material | Crosslinking metal | Metal content (wt%) | Metal content ratio | ΔR (Å) | MV (μm) |
|---|---|---|---|---|---|---|---|
| Example 1-7 | AC-7 | Na alginate | Mg/Al/Ca | 8.4 | 2.9/1/11.4 | 50 | 6 |
| Example 1-8 | AC-8 | Na alginate Na CMC | Mg/Ca | 8.0 | 1/1.8 | 34 | 5 |
| Example 1-9 | AC-9 | Na alginate Na hyaluronate | Mg/Sr | 15.5 | 1/5.8 | 50 | 30 |
| Example 1-10 | AC-10 | K alginate Na salt of fatty acid | Zn/Sr | 18.7 | 1/3.3 | 70 | 15 |
| Example 1-11 | AC-11 | Na alginate | Mg/Ca | 8.0 | 1/1.5 | 34 | 10 |
| Example 1-12 | AC-12 | Na alginate Na salt of amino acid | Mg/Sr | 13.4 | 1/3.2 | 50 | 8 |
| Comparative Example 1-1 | BC-1 | Na alginate | none | none | none | - | 6 |
| Comparative Example 1-2 | BC-2 | Na alginate | Ca | 10.1 | Ca: 100 | 0 | 6 |
| Comparative Example 1-3 | BC-3 | Na alginate | Sr | 22.1 | Sr: 100 | 0 | 6 |
| Comparative Example 1-4 | BC-4 | methyl methacrylate | none | none | none | - | 6 |

The atomic radii of the metals that furnish the at least divalent metal cations used in the Examples and Comparative Examples are shown below. The atomic radii are the numerical values given in the Diploma Program (DP) Kagaku Shiryo-shu (the Japanese language edition, published in August 2015 and revised in May 2016, of the original English-language Chemistry data booklet published in June 2014).

[Table 2]

| Metal | Atomic radius (Å) |
|---|---|
| Mg | 140 |
| Al | 124 |
| Ca | 174 |
| Zn | 120 |
| Sr | 190 |

[2] Measurement of Basic Properties

[Examples 2-1 to 2-12, Comparative Examples 2-1 to 2-4]

The water absorption for each of Marine Biodegradable Polymer Particles AC-1 to AC-12 and Comparative Polymer Particles BC-1 to BC-4 were measured by the following method, and evaluations of their heat resistances, chemical resistances and hot chemical resistances were carried out as described below.

[Measurement of Water Absorption]

Each type of particle, in an amount of 1 gram, was placed in a 500 mL beaker, following which 200 mL of deionized water was added and 30 minutes of suspension stirring (150 rpm, 25°C) was carried out. The beaker contents were then transferred to a 500 mL centrifuge tube and 30 minutes of centrifugation at 2,000G was carried out using a centrifuge (himac CR20GII, from Koki Holdings Co., Ltd.). Following centrifugation, the supernatant was gently discarded, the specimen was removed from the centrifuge tube and the weight was measured ($W_w$). The specimen was then dried to a constant weight in a 105°C drying oven and the dry weight was measured ($D_w$). The water absorption was calculated using the following formula. The results are shown in Table 3.

$$\text{Water absorption (mL/100 g)} = [(W_w - D_w)/D_w] \times 100$$

[Evaluation of Heat Resistance]

The particles, in an amount of 0.5 g, were placed in an aluminum petri dish and heated at 180°C for 2 hours in a drying oven, following which they were visually checked for melting and the shapes were checked by scanning electron microscopy (SEM). The heat resistance was evaluated according to the following criteria. The results are shown in Table 3.

[Evaluation Criteria]

| | | |
|---|---|---|
| Visual: | ○: | No major change |
| | △: | Some melting |
| | ×: | Melted |
| SEM: | 1: | Particle shapes are those of produced particles |
| | 2: | Particle shapes are maintained, but surface melting is apparent in places |
| | 3: | Partially melted, partial retention of particle shapes |
| | 4: | No particle shapes (completely melted) |

[Evaluation of Chemical Resistance]

One gram of each type of particle and 99 g of the solvent shown in Table 3 were placed in a 300 mL flask (particle content, 1 wt%) and stirred for 2 hours at room temperature (25°C), following which the dispersed state of the particles was visually checked, the particle shapes were checked by SEM, and the chemical resistance was evaluated according to the criteria below. The results are shown in Table 4.

[Evaluation of Hot Chemical Resistance]

One gram of each type of particle and 99 g of the solvent shown in Table 3 were placed in a 300 mL flask (particle content, 1 wt%) and stirred for 2 hours at 70°C, following which the dispersed state of the particles was visually checked, the particle shapes were checked by SEM, and the hot chemical resistance evaluation was evaluated according to the following criteria. The results are shown in Table 4.

[Evaluation Criteria]

◎: In direct visual examination, particles are dispersed; in SEM examination, particles are in shape of produced particles

○: In direct visual examination, particles are dispersed; in SEM examination, particle shapes are maintained, with surface melting apparent in places

△: In direct visual examination, particles are partially dispersed; in SEM examination, particles are deformed

×: In direct visual examination, particles have dissolved; in SEM examination, no particle shapes remain

[Table 3]

| | Particles | Water absorption (mL/100 g) | Heat resistance (visual/SEM) | Visible light/UV transmittance (%) | | |
|---|---|---|---|---|---|---|
| | | | | 560 nm | 400 nm | 320 nm |
| Example 2-1 | AC-1 | 118 | ○/1 | 2.3 | 2.6 | 3.0 |
| Example 2-2 | AC-2 | 122 | ○/1 | 2.8 | 3.3 | 3.9 |
| Example 2-3 | AC-3 | 106 | ○/1 | 2.0 | 2.1 | 2.2 |
| Example 2-4 | AC-4 | 132 | ○/1 | 1.7 | 1.9 | 2.1 |
| Example 2-5 | AC-5 | 115 | ○/1 | 1.9 | 2.1 | 2.3 |
| Example 2-6 | AC-6 | 102 | ○/1 | 2.2 | 2.3 | 2.5 |
| Example 2-7 | AC-7 | 108 | ○/1 | 1.9 | 2.1 | 2.4 |
| Example 2-8 | AC-8 | 98 | ○/1 | 1.8 | 2.0 | 2.2 |
| Example 2-9 | AC-9 | 101 | ○/1 | 4.0 | 5.3 | 6.0 |
| Example 2-10 | AC-10 | 34 | ○/1 | 3.0 | 3.8 | 4.6 |
| Example 2-11 | AC-11 | 120 | ○/1 | 2.5 | 2.8 | 3.1 |
| Example 2-12 | AC-12 | 26 | ○/1 | 1.8 | 2.0 | 2.2 |
| Comparative Example 2-1 | BC-1 | dissolved | ○/2 | - | - | - |
| Comparative Example 2-2 | BC-2 | 126 | ○/1 | 5.6 | 6.6 | 7.4 |
| Comparative Example 2-3 | BC-3 | 130 | ○/1 | 6.1 | 7.2 | 7.9 |
| Comparative Example 2-4 | BC-4 | 35 | ×/4 | 8.7 | 10.6 | 11.2 |

[Table 4]

| | Particles | Water | | Toluene | | Ethyl acetate | | DPG | | Ethanol | |
|---|---|---|---|---|---|---|---|---|---|---|---|
| | | Room temp. | 70°C | Room temp. | 70°C | Room temp. | 70°C | Room temp. | 70°C | Room temp. | 70°C |
| Example 2-1 | AC-1 | ◎ | ◎ | ◎ | ◎ | ◎ | ◎ | ◎ | ◎ | ◎ | ◎ |
| Example 2-2 | AC-2 | ◎ | ◎ | ◎ | ◎ | ◎ | ◎ | ◎ | ◎ | ◎ | ◎ |
| Example 2-3 | AC-3 | ◎ | ◎ | ◎ | ◎ | ◎ | ◎ | ◎ | ◎ | ◎ | ◎ |
| Example 2-4 | AC-4 | ◎ | ◎ | ◎ | ◎ | ◎ | ◎ | ◎ | ◎ | ◎ | ◎ |
| Example 2-5 | AC-5 | ◎ | ◎ | ◎ | ◎ | ◎ | ◎ | ◎ | ◎ | ◎ | ◎ |
| Example 2-6 | AC-6 | ◎ | ◎ | ◎ | ◎ | ◎ | ◎ | ◎ | ◎ | ◎ | ◎ |
| Example 2-7 | AC-7 | ◎ | ◎ | ◎ | ◎ | ◎ | ◎ | ◎ | ◎ | ◎ | ◎ |
| Example 2-8 | AC-8 | ◎ | ◎ | ◎ | ◎ | ◎ | ◎ | ◎ | ◎ | ◎ | ◎ |
| Example 2-9 | AC-9 | ◎ | ◎ | ◎ | ◎ | ◎ | ◎ | ◎ | ◎ | ◎ | ◎ |
| Example 2-10 | AC-10 | ◎ | ◎ | ◎ | ◎ | ◎ | ◎ | ◎ | ◎ | ◎ | ◎ |
| Example 2-11 | AC-11 | ◎ | ◎ | ◎ | ◎ | ◎ | ◎ | ◎ | ◎ | ◎ | ◎ |
| Example 2-12 | AC-12 | ◎ | ◎ | ◎ | ◎ | ◎ | ◎ | ◎ | ◎ | ◎ | ◎ |
| Comparative Example 2-1 | BC-1 | × | × | ◎ | ◎ | ◎ | ◎ | ◎ | ◎ | ◎ | ○ |
| Comparative Example 2-2 | BC-2 | ◎ | ◎ | ◎ | ◎ | ◎ | ◎ | ◎ | ◎ | ◎ | ◎ |

(continued)

| | Particles | Water | | Toluene | | Ethyl acetate | | DPG | | Ethanol | |
|---|---|---|---|---|---|---|---|---|---|---|---|
| | | Room temp. | 70°C | Room temp. | 70°C | Room temp. | 70°C | Room temp. | 70°C | Room temp. | 70°C |
| Comparative Example 2-3 | BC-3 | ◎ | ◎ | ◎ | ◎ | ◎ | ◎ | ◎ | ◎ | ◎ | ◎ |
| Comparative Example 2-4 | BC-4 | ○ | ○ | × | × | × | × | Δ | × | ○ | ○ |

[3] Evaluation of Particle Softness (Evaluation of Compressive Strength)

[Examples 3-1 to 3-12, Comparative Examples 3-1 to 3-4]

The 10% compressive strength $K_{10}$ at 10% displacement of the particle diameter for Marine Biodegradable Polymer Particles AC-1 to AC-12 and Comparative Polymer Particles BC-1 to BC-4 was evaluated (measurement temperature, 20°C) using the MCT-W201 Micro-Compression Tester (from Shimadzu Corporation). The results are shown in Table 5.

[Table 5]

| | Particles | 10% compressive strength $K_{10}$ (MPa) |
|---|---|---|
| Example 3-1 | AC-1 | 80 |
| Example 3-2 | AC-2 | 135 |
| Example 3-3 | AC-3 | 70 |
| Example 3-4 | AC-4 | 75 |
| Example 3-5 | AC-5 | 55 |
| Example 3-6 | AC-6 | 120 |
| Example 3-7 | AC-7 | 135 |
| Example 3-8 | AC-8 | 90 |
| Example 3-9 | AC-9 | 140 |
| Example 3-10 | AC-10 | 75 |
| Example 3-11 | AC-11 | 115 |
| Example 3-12 | AC-12 | 85 |
| Comparative Example 3-1 | BC-1 | not measurable |
| Comparative Example 3-2 | BC-2 | 380 |
| Comparative Example 3-3 | BC-3 | 320 |
| Comparative Example 3-4 | BC-4 | 530 |

[4] Sensory Tests and Evaluation of Adhesion

[Examples 4-1 to 4-12, Comparative Examples 4-1 to 4-4]

Marine Biodegradable Polymer Particles AC-1 to AC-12 and Comparative Polymer Particles BC-1 to BC-4 were evaluated for skin feel, slip characteristics and particle adhesion by the methods described below. The results are shown in Table 6.

(1) Skin Feel

The feel of each type of particle when spread over the skin was evaluated based on the criteria shown below.

(2) Slip Characteristics

One gram of each type of particle was placed on black synthetic leather and the length when spread by finger was evaluated based on the criteria shown below.

(3) Particle Adhesion

One gram of each type of particle was placed on black synthetic leather and uniformly spread with a powder puff, following which the leather was struck three times and the amount of particles remaining was examined with a digital microscope (VHX200, from Keyence Corporation) and evaluated based on the criteria shown below.

[Evaluation Criteria]

◎: Excellent
○: Good
△: Standard
×: Unacceptable

[Table 6]

|  | Particles | MV (μm) | Skin feel | Slip characteristics | Particle adhesion |
|---|---|---|---|---|---|
| Example 4-1 | AC-1 | 6 | ○ | ◎ | ○ |
| Example 4-2 | AC-2 | 6 | △ | ○ | ○ |
| Example 4-3 | AC-3 | 6 | ◎ | ○ | ○ |
| Example 4-4 | AC-4 | 6 | ◎ | ○ | ◎ |
| Example 4-5 | AC-5 | 6 | ◎ | ◎ | ◎ |
| Example 4-6 | AC-6 | 6 | ○ | ◎ | ○ |
| Example 4-7 | AC-7 | 6 | ○ | ○ | ○ |
| Example 4-8 | AC-8 | 5 | ○ | ○ | ○ |
| Example 4-9 | AC-9 | 30 | ◎ | ○ | △ |
| Example 4-10 | AC-10 | 15 | ◎ | ○ | ○ |
| Example 4-11 | AC-11 | 10 | ○ | ○ | ○ |
| Example 4-12 | AC-12 | 8 | ○ | △ | ◎ |
| Comparative Example 4-1 | BC-1 | 6 | △ | △ | ○ |
| Comparative Example 4-2 | BC-2 | 6 | △ | △ | ○ |
| Comparative Example 4-3 | BC-3 | 6 | △ | △ | ○ |
| Comparative Example 4-4 | BC-4 | 6 | ○ | ○ | ○ |

In terms of the skin feel and the slip characteristics, Marine Biodegradable Polymer Particles AC-1 to AC-12 were at least comparable to Polymer Particles BC-4, and they were able to maintain the same level of particle adhesion at the same particle diameter. Also, a tendency was observed for the skin feel and the slip characteristics to become better as the maximum difference in the sizes of the metal cations becomes larger. On the other hand, the Uncrosslinked Particles BC-1, with worsening of the moisture absorption by the base particles, tended to have a diminished skin feel and lower slip characteristics. Also, in the case of Polymer Particles BC-2 and BC-3, which were crosslinked with a single type of ion, the skin feel and slip characteristics tended to diminish somewhat due to both high crosslinkability arising from an egg-box structure and moisture absorption.

[5] Test of Solubility in Aqueous Sodium Chloride Solution (Simulated Seawater Solubility Test)

[Examples 5-1 to 5-12, Comparative Examples 5-1 to 5-4]

Marine Biodegradable Polymer Particles AC-1 to AC-12 and Comparative Polymer Particles BC-1 to BC-4 were dispersed in water or in a 3 wt% aqueous sodium chloride solution to a concentration of 0.1 wt% in each case, and solubility tests were carried out.

(1) Appearance:    The condition when 72 hours and 240 hours had elapsed following dispersion was visually checked.

(2) Shape:    Changes in shape when 72 hours and 240 hours had elapsed following dispersion in the aqueous sodium chloride solution were ascertained by measuring the particle size distribution.

The results are shown in Table 7.

[Table 7]

| | Particles | | Appearance | | Shape |
| | | | Water | Aqueous sodium chloride solution | |
|---|---|---|---|---|---|
| Example 5-1 | AC-1 | after 72 hrs | cloudy | clear | changed |
| | | after 240 hrs | cloudy | clear | changed |
| Example 5-2 | AC-2 | after 72 hrs | cloudy | clear | changed |
| | | after 240 hrs | cloudy | clear | changed |
| Example 5-3 | AC-3 | after 72 hrs | cloudy | clear | changed |
| | | after 240 hrs | cloudy | clear | changed |
| Example 5-4 | AC-4 | after 72 hrs | cloudy | clear | changed |
| | | after 240 hrs | cloudy | clear | changed |
| Example 5-5 | AC-5 | after 72 hrs | cloudy | clear | changed |
| | | after 240 hrs | cloudy | clear | changed |
| Example 5-6 | AC-6 | after 72 hrs | cloudy | clear | changed |
| | | after 240 hrs | cloudy | clear | changed |
| Example 5-7 | AC-7 | after 72 hrs | cloudy | clear | changed |
| | | after 240 hrs | cloudy | clear | changed |
| Example 5-8 | AC-8 | after 72 hrs | cloudy | clear | changed |
| | | after 240 hrs | cloudy | clear | changed |
| Example 5-9 | AC-9 | after 72 hrs | cloudy | clear | changed |
| | | after 240 hrs | cloudy | clear | changed |
| Example 5-10 | AC-10 | after 72 hrs | cloudy | clear | changed |
| | | after 240 hrs | cloudy | clear | changed |
| Example 5-11 | AC-11 | after 72 hrs | cloudy | clear | changed |
| | | after 240 hrs | cloudy | clear | changed |
| Example 5-12 | AC-12 | after 72 hrs | cloudy | clear | changed |
| | | after 240 hrs | cloudy | clear | changed |
| Comparative Example 5-1 | BC-1 | after 72 hrs | clear | clear | changed |
| | | after 240 hrs | clear | clear | changed |
| Comparative Example 5-2 | BC-2 | after 72 hrs | cloudy | clear | changed |
| | | after 240 hrs | cloudy | clear | changed |

(continued)

| | Particles | | Appearance | | Shape |
|---|---|---|---|---|---|
| | | | Water | Aqueous sodium chloride solution | |
| Comparative Example 5-3 | BC-3 | after 72 hrs | cloudy | clear | changed |
| | | after 240 hrs | cloudy | clear | changed |
| Comparative Example 5-4 | BC-4 | after 72 hrs | cloudy | cloudy | unchanged |
| | | after 240 hrs | cloudy | cloudy | unchanged |

Although Marine Biodegradable Polymer Particles AC-1 to AC-12 have a structure which, due to ionic crosslinking, does not dissolve in water, in aqueous sodium chloride solution dissolution tests, they exhibit solubilities comparable to those of Comparative Polymer Particles BC-2 and BC-3, demonstrating that, even in structures which use a plurality of at least divalent metal cations, there is no change in solubility. On the other hand, Comparative Polymer Particles BC-1 which do not have a crosslinked structure were confirmed to dissolve in both water and the aqueous sodium chloride solution. Also, Comparative Polymer Particles BC-4, which are general-purpose polymer particles, did not dissolve in either.

[6] Evaluation of Light Diffusivity

[Examples 6-1 to 6-12, Comparative Examples 6-1 to 6-4]

Aqueous dispersions (0.1 wt%) of the respective particles were prepared in 20 mL sample vials. The aqueous dispersions were then poured into respective quartz cells and, using a UV-visible spectrophotometer (UV-2450 from JASCO Corporation), transmittance spectroscopy was carried out using visible light having a wavelength of 560 nm and UV having wavelengths of 400 nm and 320 nm. The results are shown in Table 8.

[Table 8]

| | Particles | Visible/UV transmittance (%) | | |
|---|---|---|---|---|
| | | 560 nm | 400 nm | 320 nm |
| Example 6-1 | AC-1 | 2.3 | 2.6 | 3.0 |
| Example 6-2 | AC-2 | 2.8 | 3.3 | 3.9 |
| Example 6-3 | AC-3 | 2.0 | 2.1 | 2.2 |
| Example 6-4 | AC-4 | 1.7 | 1.9 | 2.1 |
| Example 6-5 | AC-5 | 1.9 | 2.1 | 2.3 |
| Example 6-6 | AC-6 | 2.2 | 2.3 | 2.5 |
| Example 6-7 | AC-7 | 1.9 | 2.1 | 2.4 |
| Example 6-8 | AC-8 | 1.8 | 2.0 | 2.2 |
| Example 6-9 | AC-9 | 4.0 | 5.3 | 6.0 |
| Example 6-10 | AC-10 | 3.0 | 3.8 | 4.6 |
| Example 6-11 | AC-11 | 2.5 | 2.8 | 3.1 |
| Example 6-12 | AC-12 | 1.8 | 2.0 | 2.2 |
| Comparative Example 6-1 | BC-1 | - | - | - |
| Comparative Example 6-2 | BC-2 | 5.6 | 6.6 | 7.4 |
| Comparative Example 6-3 | BC-3 | 6.1 | 7.2 | 7.9 |
| Comparative Example 6-4 | BC-4 | 8.7 | 10.6 | 11.2 |

Based on the results shown in Table 8, because the transmitted light decreases in the UV region, it was confirmed that the marine biodegradable polymer particles of the invention clearly have a high UV-scattering effect. Also, given that the light-scattering effect is high even in the visible light region, it was confirmed that these particles also have a high hiding power.

[7] Production and Evaluation of Optical Measurement Sheets

[Examples 7-1 to 7-12, Comparative Examples 7-1 to 7-4]

Compositions for optical measurement sheets were prepared by adding 35.0 g of a binder resin (a PVA resin from Kuraray Co., Ltd.) and 75.0 g of purified water to 15.0 g of, respectively, Marine Biodegradable Polymer Particles AC-1 to AC-12 and Comparative Polymer Particles BC-1 to BC-4 and mixing the ingredients together. Each of the resulting compositions was then coated onto one side of a 100 μm thick PET film (E-5000, from Toyobo Co., Ltd.) using a commercial bar coater. After coating, a drying oven was set to 60°C and hot-air drying was carried out for 20 minutes, thereby producing Optical Sheets 1 to 16 having a coated layer thickness of 40 μm.

Using an automated goniophotometer (GP-200, from Murakami Color Research Laboratory Co., Ltd.), a fixed amount of light was irradiated onto each of Optical Sheets 1 to 16 at an incident angle of 45° and the light scattering distribution of the reflected light was measured. The light-diffusing ability of each sheet was evaluated according to the following criteria. The results are shown in Table 9.

[Evaluation Criteria]

Optical Sheet 16 (Polymer Particles BC-4) was used as the reference.

A: Good diffusivity
B: Substantially the same diffusivity
C: Poor diffusivity

[Table 9]

| | Optical sheet | Particles | Diffusivity |
|---|---|---|---|
| Example 7-1 | 1 | AC-1 | A |
| Example 7-2 | 2 | AC-2 | B |
| Example 7-3 | 3 | AC-3 | A |
| Example 7-4 | 4 | AC-4 | A |
| Example 7-5 | 5 | AC-5 | A |
| Example 7-6 | 6 | AC-6 | A |
| Example 7-7 | 7 | AC-7 | A |
| Example 7-8 | 8 | AC-8 | A |
| Example 7-9 | 9 | AC-9 | B |
| Example 7-10 | 10 | AC-10 | A |
| Example 7-11 | 11 | AC-11 | A |
| Example 7-12 | 12 | AC-12 | A |
| Comparative Example 7-1 | 13 | BC-1 | dissolved, viscosity rose, could not form a sheet |
| Comparative Example 7-2 | 14 | BC-2 | B |
| Comparative Example 7-3 | 15 | BC-3 | C |
| Comparative Example 7-4 | 16 | BC-4 | reference |

In the particles that were crosslink-treated with at least divalent metal cations, it was confirmed that a performance equal to or better than that of the Optical Sheet 16 produced using the general-purpose Polymer Particles BC-4 can be obtained. In particular, at the same particle diameter, superior scattering properties tended to be observed when the maximum difference in the metal cations used was larger.

[8] Evaluation of Optical Properties and Tactile Feel in Paint

[Examples 8-1 to 8-12, Comparative Examples 8-1 to 8-4]

Marine Biodegradable Polymer Particles AC-1 to AC-12 and Comparative Polymer Particles BC-1 to BC-4 were respectively added to a commercial oil-based paint (Pro Touch, from Rock Paint Co., Ltd.) to a concentration of 5 wt%, applied onto an aluminum substrate and dried, thereby forming films having a dry-film thickness of 2 $\mu$m and producing Paint Film Sheets 1 to 16.

The light reflectivity and presence/absence of gloss in the resulting paint film sheets were visually evaluated and sensory evaluations of the tactile effect were carried out. The tactile effect was evaluated by running a finger over the paint film and assessing the soft feel according to the criteria indicated below. An aluminum substrate on which a film had not been formed was used as the blank. The results are shown in Table 10.

[Evaluation Criteria]

○: soft feel is apparent
△: somewhat of a soft feel
✕ : soft feel is absent

[Table 10]

| | Paint film sheet | Particles | MV ($\mu$m) | Reflectivity (hiding power) | Gloss | Soft feel |
|---|---|---|---|---|---|---|
| Example 8-1 | 1 | AC-1 | 6 | ○ | no | ○ |
| Example 8-2 | 2 | AC-2 | 6 | ○ | no | ○ |
| Example 8-3 | 3 | AC-3 | 6 | ○ | no | ○ |
| Example 8-4 | 4 | AC-4 | 6 | ○ | no | ○ |
| Example 8-5 | 5 | AC-5 | 6 | ○ | no | ○ |
| Example 8-6 | 6 | AC-6 | 6 | ○ | no | ○ |
| Example 8-7 | 7 | AC-7 | 6 | ○ | no | ○ |
| Example 8-8 | 8 | AC-8 | 5 | ○ | no | ○ |
| Example 8-9 | 9 | AC-9 | 30 | △ | some gloss | ○ |
| Example 8-10 | 10 | AC-10 | 15 | ○ | some gloss | ○ |
| Example 8-11 | 11 | AC-11 | 10 | ○ | no | ○ |
| Example 8-12 | 12 | AC-12 | 8 | ○ | some gloss | ○ |
| Comparative Example8-1 | 13 | BC-1 | 6 | △ | some gloss | △ |
| Comparative Example8-2 | 14 | BC-2 | 6 | △ | no | △ |
| Comparative Example8-3 | 15 | BC-3 | 6 | △ | no | △ |
| Comparative Example8-4 | 16 | BC-4 | 6 | dissolved | dissolved | ✕ |
| Blank | Al substrate | - | - | glossy | yes | ✕ |

Paint Film Sheets 1 to 12 containing Marine Biodegradable Polymer Particles AC-1 to AC-12 had hiding powers equal to or better than Paint Film Sheets 13 to 15, indicating that these particles can be used as delusterants or as texture modifying agents.

[9] Evaluation of Seawater Biodegradability

[Examples 9-1 to 9-12, Comparative Examples 9-1 to 9-4]

Seawater biodegradation tests were performed by the following method on Marine Biodegradable Polymer Particles AC-1 to AC-12 and Comparative Polymer Particles BC-1 to BC-4 using microcrystalline cellulose (Avicel PH-101, from Sigma-Aldrich Co.) as the control material. The results are shown in Table 11.

<Test Method and Conditions>

| Method for Measuring Degree of Biodegradation: | The oxygen consumption was measured with a closedrespirometer (see ASTM D6691) |
|---|---|
| Incubation Temperature: | 30±1°C, in the dark |

$$\text{Degree of Biodegradation (\%): } [(BOD_O - BOD_B)/ThOD] \times 100$$

BODo: Biochemical oxygen demand in test or inoculum activity verification (measured value in mg)

$BOD_B$: Average biochemical oxygen demand in blank test (measured value in mg)

ThOD: Theoretical oxygen demand required when test material or control material has completely oxidized (calculated value in mg)

| Seawater: | collected from Tokyo Bay (Port of Chiba in Chiba Prefecture)) |
|---|---|
| Number of Samples: | The average value for three samples (n =3) was used for each type of particle. |

[Evaluation Criteria]

Evaluation was carried out by setting the incubation period to 60 days and calculating the relative degree of biodegradation (%), defined here as the ratio of the maximum degree of biodegradation by each type of particle to the degree of biodegradation (absolute degree of biodegradation) of microcrystalline cellulose as the control sample. The absolute degree of degradation by cellulose as the control sample was 66%.

◎: Marine biodegradability is comparable with that of cellulose (relative degree of biodegradation ≥ 90%)

○: Has marine biodegradability (relative degree of biodegradation, 60 to 90%)

△: Possibility of having marine biodegradability over medium-to-long term (relative degree of biodegradation, from 10 to <60%)

×: High possibility that substantially no marine biodegradability is observable (relative degree of biodegradation <10%)

[Table 11]

| | Particles | Degree of biodegradation relative to cellulose (%) | Evaluation of seawater biodegradability |
|---|---|---|---|
| Example 9-1 | AC-1 | 105 | ◎ |
| Example 9-2 | AC-2 | 99 | ◎ |
| Example 9-3 | AC-3 | 98 | ◎ |
| Example 9-4 | AC-4 | 100 | ◎ |

(continued)

|  | Particles | Degree of biodegradation relative to cellulose (%) | Evaluation of seawater biodegradability |
|---|---|---|---|
| Example 9-5 | AC-5 | 99 | ◎ |
| Example 9-6 | AC-6 | 98 | ◎ |
| Example 9-7 | AC-7 | 88 | ○ |
| Example 9-8 | AC-8 | 100 | ◎ |
| Example 9-9 | AC-9 | 92 | ◎ |
| Example 9-10 | AC-10 | 94 | ◎ |
| Example 9-11 | AC-11 | 100 | ◎ |
| Example 9-12 | AC-12 | 92 | ◎ |
| Comparative Example 9-1 | BC-1 | 110 | ◎ |
| Comparative Example 9-2 | BC-2 | 105 | ◎ |
| Comparative Example 9-3 | BC-3 | 101 | ◎ |
| Comparative Example 9-4 | BC-4 | 0.3 | × |
| Control sample | cellulose | - | 66% (absolute degree of degradation) |

The results shown in Table 11 demonstrate that the particles of the invention have a good biodegradability in seawater.

[10] Preparation and Evaluation of Skin Cleansing Compositions

[Examples 10-1 to 10-3, Comparative Example 10-1]

Using Marine Biodegradable Polymer Particles AC-1, AC-9 and AC-10 and Comparative Polymer Particles BC-2, skin cleansing compositions (Cleansing Compositions 1 to 4) were produced according to the formulations shown in Table 12 below.

[Table 12]

| Ingredients | | Example 10-1 | Example 10-2 | Example 10-3 | Comparative Example 10-1 |
|---|---|---|---|---|---|
| | | Cleansing Composition 1 | Cleansing Composition 2 | Cleansing Composition 3 | Cleansing Composition 4 |
| Ingredient amounts (g) | Stearic acid | 2.0 | 2.0 | 2.0 | 2.0 |
| | Palmitic acid | 4.0 | 4.0 | 4.0 | 4.0 |
| | Myristic acid | 10.0 | 10.0 | 10.0 | 10.0 |
| | Lauric acid | 3.0 | 3.0 | 3.0 | 3.0 |
| | Lauramidopropylamine oxide | 20.0 | 20.0 | 20.0 | 20.0 |
| | Polypropylene glycol (400) | 2.0 | 2.0 | 2.0 | 2.0 |
| | Triethanolamine | 14.0 | 14.0 | 14.0 | 14.0 |
| | Ethylene glycol distearate | 1.0 | 1.0 | 1.0 | 1.0 |
| | Purified water | 41.0 | 41.0 | 41.0 | 41.0 |
| | Particles AC-1 | 3.0 | - | - | - |
| | Particles AC-9 | - | 3.0 | - | - |
| | Particles AC-10 | - | - | 3.0 | - |
| | Particles BC-2 | - | - | - | 3.0 |

Evaluations were carried out by the following methods on each of the cleansing compositions prepared. The results are shown in Table 13.

Ten people were selected as panelists and usage tests in which the skin cleansing compositions were used to cleanse the face were carried out. The six qualities of "Feeling on Use 1," "Feeling on Use 2," "Latherability," "Skin Cleansing and Exfoliating Effects," "Massaging Effects" and "Irritancy" were evaluated according to the criteria shown below, based on which each composition was rated overall as a face scrub.

| | |
|---|---|
| • Feeling on Use 1: | Pleasantness of application and agreeability with skin during use |
| • Feeling on Use 2: | Absence of feeling of residual face scrub and skin tightness after rinsing off cleanser |
| • Latherability: | Ease of foam production and foam longevity when cleanser is used |
| • Skin Cleansing andExfoliating Effects: | Degree to which makeup comes off following use |
| • Massaging Effects: | Are massaging effects apparent after washing, such as resolution of dark patches on skin, improvement in facial complexion and promotion of blood circulation? |
| • Irritancy: | Absence of redness, tingling, etc. after rinsing off cleanser |

[Evaluation Criteria for Each Quality]

◎: Clearly effective (good feeling) [rated highly by 8 or more panelists]
○: Found to be effective (somewhat good feeling) [rated highly by 6 or 7 panelists]
□: Found to be effective (somewhat good feeling) [rated highly by 4 or 5 panelists]
△: Not very effective (somewhat poor feeling) [rated highly by 2 or 3 panelists]
×: Ineffective (poor feeling) [rated highly by no more than 1 panelist]

[Scoring Criteria]

◎: 8 points
○: 6 points
□: 4 points
△: 2 points
×: 0 points

[Overall Rating]

A: 38 points or more

B: 30 to 37 points

C: 22 to 29 points

D: 21 points or less

[Table 13]

|  | Example 10-1 | Example 10-2 | Example 10-3 | Comparative Example 10-1 |
|---|---|---|---|---|
|  | Cleansing Composition 1 | Cleansing Composition 2 | Cleansing Composition 3 | Cleansing Composition 4 |
| Feeling on Use 1 | ○ | ◎ | ○ | □ |
| Feeling on Use 2 | ○ | ○ | ○ | ○ |
| Latherability | ○ | □ | ◎ | □ |
| Skin Cleansing and Exfoliating Effect | ○ | ◎ | ○ | ○ |
| Massaging Effect | □ | ○ | ○ | □ |
| Irritancy | ○ | ○ | ○ | □ |
| Score | 34 | 38 | 38 | 28 |
| Overall evaluation as face scrub | B | A | A | C |

As shown in Table 13, in terms of feeling and low irritancy as well, the particles of the invention are also useful as an additive (basic ingredient) in body cleansing compositions.

[11] Confirmation of Utility in Skin Care Preparations

[Reference Examples 1 to 14]

Makeup compositions (Foundations 1 to 14) containing Marine Biodegradable Polymer Particles AC-1 to AC-12 and Comparative Polymers BC-2 and BC-4 were prepared according to the formulations shown in Table 14 below.

[Table 14]

| Ingredients | | Reference Examples 1 to 14 |
|---|---|---|
| | | Foundations 1 to 14 |
| Ingredient amounts (g) | Red iron oxide | 0.4 |
| | Yellow iron oxide | 1.0 |
| | Black iron oxide | 0.2 |
| | Titanium oxide | 7.0 |
| | Zinc oxide | 3.0 |
| | Silicone-treated large-particle-size titanium oxide | 3.0 |
| | Lauroyl lysine powder | 14.0 |
| | Titanium mica | 4.0 |
| | Talc | 37.97 |
| | Methyl phenyl polysiloxane | 2.0 |
| | Crystalline cellulose | 5.0 |
| | Cornstarch | 10.0 |
| | Methylparaben | 0.1 |
| | Sodium dehydroacetate | 0.1 |
| | Liquid paraffin | 1.5 |
| | Butylene glycol | 0.5 |
| | Coix seed extract | 0.1 |
| | Carrot extract | 0.1 |
| | Ubiquinone | 0.03 |
| | Particles AC-1 to AC-12, BC-2 and BC-4 | 10.0 |

It was possible to formulate Marine Biodegradable Polymer Particles AC-1 to AC-12 of the invention as cosmetic ingredients in the same way as the compounding ingredients BC-2 and BC-4 which are widely used in cosmetics and with no loss of performance, demonstrating that materials using the inventive particles can be employed in cosmetics in much the same way as conventional materials.

As explained above, given that marine biodegradable polymer particles composed primarily of a polymer compound in which water-soluble polymeric polyvalent anions derived from alginic acid and having monovalent anionic substituents are crosslinked through two or more types of at least divalent metal cations can be stably and efficiently produced with little extraneous matter such as agglomerates, and that crosslinkable particles having heat resistance and resistance to (hot) chemicals also can be stably produced, use in a variety of applications is possible.

Moreover, the marine biodegradable polymer particles of the invention are particles which are size-controlled and environmentally friendly. Hence, such particles can be put to effective practical use, particularly as a natural polymer-derived ingredient having marine biodegradability that is capable of serving as a countermeasure to marine contamination, in applications for which environmental compatibility may be required, such as paints, inks, molded articles and cosmetics.

## Claims

1. Marine biodegradable polymer particles comprising a polymer compound in which water-soluble polymeric polyvalent anions having monovalent anionic substituents are crosslinked through two or more types of at least divalent metal cations, the water-soluble polymeric polyvalent anions including at least one anion derived from alginic acid, wherein the marine biodegradable polymer particles satisfy conditions (1) to (4) below:

   (1) the content of the at least divalent metal cations included in the particles is from 3 to 30 wt%;
   (2) the difference between the largest and smallest atomic radii of the metal elements making up the at least divalent metal cations is 15 Å or more;
   (3) the content of the at least divalent metal cations derived from metal elements having an atomic radius of 150 Å or more is at least 25 wt% of all the at least divalent metal cations; and
   (4) the particles have a water absorption of less than 300 mL/100 g.

2. The marine biodegradable polymer particles of claim 1, wherein the monovalent anionic substituents are carboxylate anions.

3. The marine biodegradable polymer particles of claim 1, wherein the water-soluble polymeric polyvalent anions are derived from a polysaccharide.

4. The marine biodegradable polymer particles of claim 1, wherein the difference between the largest and smallest atomic radii of the metal elements making up the at least divalent metal cations is 100 Å or less.

5. The marine biodegradable polymer particles of claim 1, wherein the atomic radii of the metal elements making up the at least divalent metal cations are from 80 to 220 Å.

6. The marine biodegradable polymer particles of claim 1, wherein at least one of the at least divalent metal cations is a beryllium, magnesium, calcium, strontium, barium, zinc or aluminum ion.

7. The marine biodegradable polymer particles of claim 6, wherein at least one of the at least divalent metal cations is a calcium or strontium ion.

8. The marine biodegradable polymer particles of claim 1 which further satisfy condition (5) below:
   (5) the 10% compressive strength $K_{10}$ at 10% displacement of the particle diameter is from 1 to 1,000 MPa.

9. The marine biodegradable polymer particles of claim 1 which further satisfy condition (6) below:
   (6) the melting temperature is at least 150°C.

10. A method for producing marine biodegradable polymer particles, comprising the step of crosslinking water-soluble polymeric polyvalent anions derived from a water-soluble anionic polymer containing at least a monovalent salt of alginic acid and having monovalent anionic substituents within:

   (A) a medium in which particles of the water-soluble anionic polymer have been dispersed to a concentration of at least 5 wt%,
   (B) a medium in which, using water and an oily medium, the water-soluble anionic polymer has been suspended or emulsified in water to a concentration of at least 5 wt%, or
   (C) a medium in which the water-soluble anionic polymer has been hydrophilized or dissolved to a concentration of at least 5 wt%

   by using as crosslinking agents two or more salt compounds containing at least divalent metal cations;
   wherein the crosslinking reaction is carried out by adding the two or more salt compounds a plurality of times in divided portions, such that the difference between the largest and smallest atomic radii of the metal elements making up the at least divalent metal cations included in the salt compound is 15 Å or more, and the content of the at least divalent metal cations derived from metal elements having an atomic radius of 150 Å or more is at least 25 wt% of all the at least divalent metal cations.

11. The method for producing marine biodegradable polymer particles of claim 10 wherein, in the crosslinking step, the crosslinking reaction is carried out by independently adding at least one of the two or more salt compounds.

12. An ultraviolet scattering agent comprising the marine biodegradable polymer particles of any one of claims 1 to 9.

13. A marine biodegradable additive comprising the marine biodegradable polymer particles of any one of claims 1 to 9.

14. A personal care product comprising the marine biodegradable additive of claim 13.

15. A cosmetic comprising the marine biodegradable additive of claim 13.

16. A coating comprising the marine biodegradable additive of claim 13.

17. An ink comprising the marine biodegradable additive of claim 13.

18. A resin composition comprising the marine biodegradable additive of claim 13.

19. A formed body comprising the marine biodegradable additive of claim 13.

# INTERNATIONAL SEARCH REPORT

| International application No. |
|---|
| **PCT/JP2023/039849** |

| A. | CLASSIFICATION OF SUBJECT MATTER |
|---|---|

*C08J 3/24*(2006.01)i; *C09D 5/16*(2006.01)i; *A61Q 1/00*(2006.01)i; *A61Q 19/10*(2006.01)i; *C08K 5/09*(2006.01)i; *C08L 5/04*(2006.01)i; *C08L 101/16*(2006.01)i; *C09D 201/00*(2006.01)i; *C09D 7/65*(2018.01)i; *A61K 8/73*(2006.01)i
FI:   C08J3/24 CEP; C09D5/16; C09D7/65; C09D201/00; A61K8/73; A61Q19/10; A61Q1/00; C08L101/16; C08L5/04 ZBP; C08K5/09 ZAB

According to International Patent Classification (IPC) or to both national classification and IPC

| B. | FIELDS SEARCHED |
|---|---|

Minimum documentation searched (classification system followed by classification symbols)

C08J3/24; C09D5/16; A61Q1/00; A61Q19/10; C08K5/09; C08L5/04; C08L101/16; C09D201/00; C09D7/65; A61K8/73

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2001-64175 A (TEIKOKU SEIYAKU CO., LTD.) 13 March 2001 (2001-03-13) claims, examples | 18 |
| A | | 10-12 |
| X | JP 2021-507946 A (ASSOCIATION FOR THE ADVANCEMENT OF TISSUE ENGINEERING AND CELL BASED TECHNOLOGIES & THERAPIES (A4TEC) ASSOCIAÇÃO) 25 February 2021 (2021-02-25) claims, examples | 1-9, 18 |
| A | | 10-12 |
| X | CN 113509912 A (QINGHAI INST. SALT LAKES CAS) 19 October 2021 (2021-10-19) claims, paragraphs [0037]-[0042], examples | 1-9, 18 |
| A | | 10-12 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **21 December 2023** | **16 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
| --- |
| **PCT/JP2023/039849** |

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2004-18680 A (NEC CORPORATION) 22 January 2004 (2004-01-22) claims, paragraphs [0022]-[0026], [0045]-[0046], [0060], examples | 1-9, 18-19 |
| Y | | 1-9, 13-19 |
| A | | 10-12 |
| Y | JP 2020-79347 A (NIPPON SHOKUBAI CO., LTD.) 28 May 2020 (2020-05-28) claims, paragraphs [0015]-[0020], [0035], examples | 1-9, 13-19 |
| A | | 10-12 |
| Y | WO 2022/053553 A1 (XAMPLA LIMITED) 17 March 2022 (2022-03-17) claims, page 15, line 31 to page 16, line 13, examples | 13-17 |
| A | | 10-12 |

**INTERNATIONAL SEARCH REPORT**
Information on patent family members

International application No.

**PCT/JP2023/039849**

| Patent document cited in search report | | | Publication date (day/month/year) | Patent family member(s) | | | Publication date (day/month/year) |
|---|---|---|---|---|---|---|---|
| JP | 2001-64175 | A | 13 March 2001 | US | 6528077 | B1 | |
| | | | | claims, examples | | | |
| JP | 2021-507946 | A | 25 February 2021 | US | 2021/0000982 | A1 | |
| | | | | claims, examples | | | |
| CN | 113509912 | A | 19 October 2021 | (Family: none) | | | |
| JP | 2004-18680 | A | 22 January 2004 | (Family: none) | | | |
| JP | 2020-79347 | A | 28 May 2020 | (Family: none) | | | |
| WO | 2022/053553 | A1 | 17 March 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020125256 A
- JP 2021195321 A
- JP 2021191810 A

**Non-patent literature cited in the description**

- **SATO, T. et al.** Development of calcium alginate microparticles and expansion of their use into cosmetics. *Sen'i to Kogyo*, 1996, vol. 20 (1), 20-26
- Diploma Program (DP) Kagaku Shiryo-shu. August 2015
- Chemistry data booklet. June 2014